# EUROPEAN PATENT APPLICATION

(11) **EP 4 057 290 A1**
(43) Date of publication of application: **14.09.2022**
(21) Application number: 22161336.7
(22) Date of filing: 10.03.2022
(51) Int. Cl.: G16H 20/17, G16H 50/20, A61B 5/00, A61M 5/145

(54) **SYSTEMS AND METHODS FOR TREATING NEUROLOGICAL CONDITIONS IN PARKINSON DISEASE SUBJECTS**

(30) Priority: 12.03.2021 US 202163160289 P
(71) Applicant: Neuroderm Ltd, 7670212 Rehovot (IL)
(72) Inventor: SHOR, Eran, 7670212 Rehovot (IL); DAVID, Tamir Ben, 7670212 Rehovot (IL)
(74) Representative: HGF

(57) **Abstract**

Systems and methods are provided for treating a condition associated with a Parkinson's disease (PD) subject, for example for treating or ameliorating motor and/or nonmotor symptoms or disorders in Parkinson's disease patients. An example system may include a sleep sensing circuitry and a drug dispensing device. The sleep sensing circuitry may be used to detect a sleep pattern of a PD patient, and the drug dispensing device may be used to continuously deliver a therapeutically effective compound to the PD patient based on the sleep pattern. Operational parameters of the drug delivery device (e.g., drug delivery flow rate and drug delivery timing) may be pre-programmed prior to bedtime and/or be adjusted in real time, for example during sleep, based on the detected sleep pattern to treat a condition associated with the PD subject, for example to optimize sleep, improve sleep quality, ameliorate nonmotor disorders in the treated PD patient, etc.

## Description

### FIELD OF THE INVENTION

The present invention generally relates to systems and methods for delivering formulations/compositions/compounds (for example formulations which may be based on, or include, levodopa (LD) and/or carbidopa (CD), or a prodrug of LD and/or CD) for the treatment of (e.g., ameliorating) motor and non-motor neurological conditions (e.g., sleep disorders) in subjects with Parkinson's disease (PD). The present invention also relates to systems and methods for optimizing sleep, improving sleep quality, and ameliorating sleep disorders in PD subjects.

### BACKGROUND

PD is a chronic neurodegenerative disorder characterized by progressive loss of dopamine-producing neurons and presents with motor symptoms including, for example, slowness/bradykinesia, rigidity, tremor, muscle stiffness, dyskinesia, postural instability, etc. LD, a dopamine precursor, is currently considered to be the most effective and gold standard therapy for treating patients with PD. NeuroDerm Ltd. (a company based in Israel) has developed a proprietary liquid formulation of LD/CD for subcutaneous and continuous delivery to PD subjects by using a small, wearable, pump device.

PD patients also experience non-motor symptoms. While motor complications in PD patients result in physical disability, non-motor complications may be of equal or greater significance in some PD patients. Non-motor symptoms can include, for example, mood disorders (e.g., depression, anxiety, irritability); cognitive changes (e.g., impaired focused attention and planning, language and memory difficulties, slowing of thought, dementia); hallucinations and delusions, constipation and early satiety, pain, fatigue, vision problems, excessive sweating (especially of hands and feet, with no or little exercise); urinary urgency (frequency and incontinence); loss of sense of smell; autonomic dysfunction and various types of sleep disorders including, for example, insomnia, excessive daytime sleepiness (EDS), rapid eye movement behavior disorder (RBD), vivid dreams, talking and moving during sleep, restless legs syndrome (RLS), periodic leg movements disorder (PLMD), sleep latency, insufficient total sleep time, disorders related to sleep efficiency, sleep onset, sleep maintenance, sleep fragmentation, and altered number of sleep cycles correlated with diurnal somnolence and nocturnal psychosis. A PD patient experiencing disrupted sleep during the night, or regardless of that, may be prone to sudden unintentional sleep "attacks" (narcolepsy), causing a person involved to fall asleep unexpectedly during the daytime. Sleep disturbances such as sleep onset insomnia, sleep fragmentation, rapid eye movement sleep behavior disorder (RBD) and excessive daytime sleepiness (EDS), to name a few, are major disabling non-motor symptoms in PD patients.

Some scientific publications suggest that, at least for some PD patients, some of the non-motor symptoms described above (e.g., sleep disorders) may be side effects caused, or aggravated, by long-term use of levodopa. It would be beneficial to have a system and a method which can efficiently treat motor symptoms of PD patients while ameliorating (e.g., moderating or minimizing) non-motor symptoms (for example sleep disorders), or that can ameliorate non-motor symptoms without having an adverse effect on the motor symptom treatment.

### SUMMARY OF THE INVENTION

Systems and methods are provided for treating a condition associated with a PD subject, for example for treating or ameliorating motor and/or nonmotor symptoms or disorders in PD patients. A system may generally include a sleep sensing circuitry and a drug delivery device (e.g., pump device). The sleep sensing circuitry may be used to detect a sleep pattern of a PD patient, and the drug delivery device may be used to continuously deliver a therapeutically effective compound (e.g., a drug) to the PD patient based on the sleep pattern. Operational parameters of the drug delivery device (e.g., drug delivery flow rate and drug delivery timing) may be, for example, pre-programmed prior to bedtime and/or be adjusted in real time, for example during sleep, based on the detected sleep pattern (or based on historical sleep patterns) to treat a condition associated with the PD subject, for example to optimize sleep, improve sleep quality, ameliorate non-motor disorders in the treated PD patient, etc.

An example method for operating a therapeutic drug delivery system for treating a condition associated with a PD subject may include the steps of receiving, by the therapeutic drug delivery system, sleep data that characterizes, or includes, a chronologic sleep pattern (CSP) of a PD subject, determining values for one or more operational parameters of the therapeutic drug delivery system based on the CSP, and/or receiving said values for the one or more operational parameters of the therapeutic drug delivery system from an external source, for example from a remote computer. The method may also include the step of operating the therapeutic drug delivery system according to the received operational parameters values, or according to the determined operational parameters values, to deliver a therapeutic drug composition (or compound) to the PD subject in a drug delivery pattern ("DDP") that is advantageous in treating the condition. (a specific DDP may be used to treat a specific condition in a specific PD subject.) A similar method may be used to deliver, by the therapeutic drug delivery system, a compound for treating a neurological condition in subjects with PD.

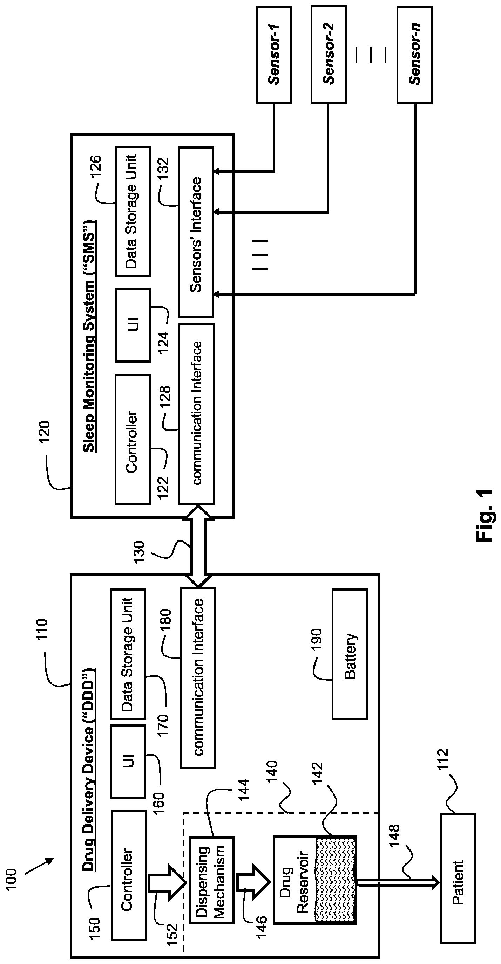

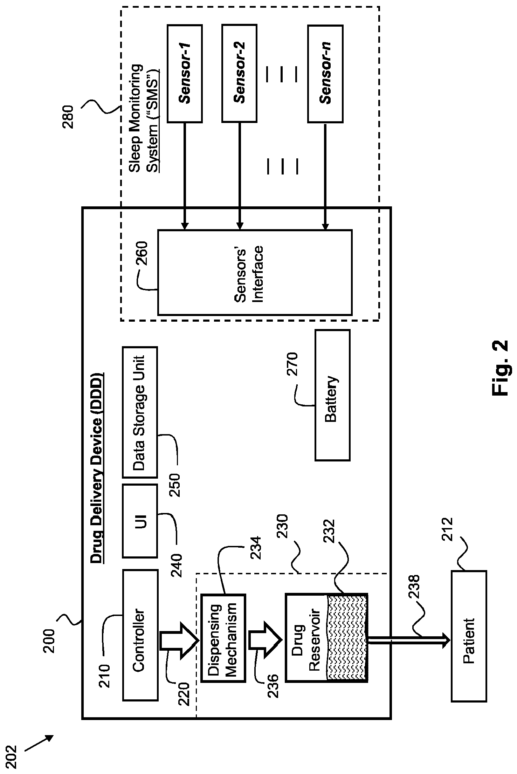

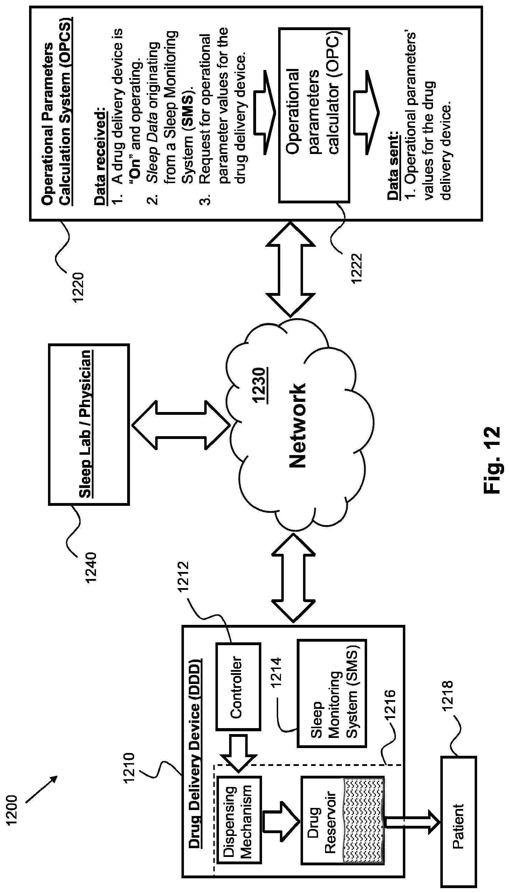

An example DDS (DDS **100,** Fig. 1; DDS **202,** Fig. 2; DDS **1200,** Fig. 12) for treating a condition associated with a subject with PD may include a sleep data collecting unit (SDCU). The SDCU may, in general, produce, collect (e.g., receive) and/or store sleep data (e.g., real time sleep data and/or historical sleep data) that is related to a PD subject, and/or sleep data (real time data and/or historical data) that is related to other PD subjects. The SDCU may be configured in a configuration that may be selected from at least one of: (i) a first configuration in which the SDCU is, or includes, a sleep monitoring system (SMS) **(120, 280),** and (ii) a second configuration in which the SDCU is, or includes, a user interface **(160, 240)** for manually uploading sleep data and/or a communication interface **(180)** for remotely receiving sleep data and/or operational parameters values for operating a drug dispensing mechanism.

The SMS may include a number **"n"** of sensors (***n***=1, 2, 3,..., etc.) that may be configured to produce signals associated, individually or collectively, with a sleep condition and/or motor activity of the PD subject. The SMS may also include a sensors interface **(132, 260)** that may be connected to the **n** sensors and convert the sensors' signals into sleep data.

The DDS may also include a drug delivery unit **(140, 230).** The drug delivery unit may include a drug reservoir **(142, 232)** that may contain therapeutic drug composition, and a dispensing mechanism **(144, 234)** for dispensing the therapeutic drug composition from the drug reservoir to the PD subject.

The system may also include a controller **(150, 210, 1212)** to control the operation of the dispensing mechanism. The controller may be configured to, among other things: (i) receive values and/or determine values, e.g., that are derived from the sleep data, for one or more drug delivery operational parameters of the dispensing mechanism, and (ii) operate the dispensing mechanism to deliver the therapeutic drug composition to the PD subject according to the values received and/or determined for the one or more operational parameters. The controller may be further configured to detect a chronologic sleep pattern (CSP) in the sleep data associated with the PD subject, and deliver the therapeutic drug composition to the subject using a drug delivery pattern (DDP) that corresponds to, is adjusted for, is adapted to, or is derived from the CSP. The operational parameters values, which the controller may use to operate the dispensing mechanism, may define the DDP. For example, the controller may adjust the operational parameters values in such a way that would cause the drug to be delivered (dispensed) according to a therapeutically desired DDP.

The treated condition may be selected from the group consisting of Parkinson symptom, motor complication, motor symptom, nonmotor symptom, and sleep disorder. The values determined for the operational parameters and the values received for the operational parameters may be optimized in terms of optimizing sleep, improving sleep quality, ameliorating a sleep disorder, and ameliorating a Parkinson symptom including tremor, shaking, slowed movement (bradykinesia), muscles rigidity, postural instability, walking/gait difficulties and dystonia; sleep duration, time interval from getting to bed to sleep onset time, number of awakenings during sleep, speed of body movement during sleep, time interval from awakening time until standing up, period of Parkinson "on" time versus "off' time during wake time, awake time period during sleep, number of body rotations during sleep, average speed of rotation of body during sleep, average time of body rotation, degree of body rotation (degrees), or any combination thereof.

### BRIEF DESCRIPTION OF THE DRAWINGS

Various exemplary embodiments and aspects are illustrated in the accompanying figures with the intent that these examples be not restrictive. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. Of the accompanying figures:
Fig. 1 shows a first configuration of a drug delivery system for treating a condition in a PD subject according to an example embodiment;
Fig. 2 shows a second configuration of a drug delivery system for treating a condition in a PD subject according to another example embodiment;
Fig. 3 shows an example hypnogram including an example chronologic sleep pattern ("CSP");
Fig. 4 schematically illustrates a drug delivery control scheme for treating a condition in the PD subject in accordance with an example embodiment;
Fig. 5 schematically illustrates a drug delivery control scheme for treating a condition in a PD subject according to another example embodiment;
Fig. 6 schematically illustrates a drug delivery control scheme for treating a condition in a PD subject according to yet another example embodiment;
Fig. 7 shows a method of operating a drug delivery system for treating a condition in a PD subject according to an example embodiment;
Fig. 8 shows a method of operating a therapeutic drug delivery device for treating a condition in a PD subject according to yet another example embodiment;
Fig. 9 shows a method of operating a therapeutic drug delivery device for treating a condition in a PD subject according to yet another example embodiment;
Fig. 10 shows a method of operating a therapeutic drug delivery device for treating a condition in a PD subject according to still another example embodiment;
Fig. 11 shows a method of operating a therapeutic drug delivery device for treating a condition in a PD subject according to an example embodiment;
Fig. 12 shows a third configuration of a drug delivery system for treating a condition in a PD subject according to yet another example embodiment; and
Fig. 13 shows a method of controlling a drug delivery control scheme in real time according to an example embodiment.

### DETAILED DESCRIPTION OF THE INVENTION

The description that follows provides various details of example embodiments. However, this description is not intended to limit the scope of the claims but instead to explain various principles of the invention and exemplary manners of practicing it.

The terms "treat," "treatment," "treating," "imporving," "ameliorating," and the like are used herein to generally refer to obtaining a desired pharmacological and/or physiological effect. The effect may be therapeutic in terms of partially or completely curing a disease and/or adverse effects or symptoms attributed to the disease. The term "treatment" as used herein covers any treatment of a disease in a mammal, particularly a human, and includes: (a) inhibiting the disease, i.e., preventing the disease from increasing in severity or scope; (b) relieving the disease, i.e., causing partial or complete amelioration or imporvment of the disease; or (c) preventing relapse of the disease, i.e., preventing the disease from returning to an active state following previous successful treatment of symptoms of the disease or treatment of the disease. A treated "condition" in a PD subjet may be, for example, any PD motor symptom and/or any non-motor symptom a PD subject may experience. Treating a non-motor symptom in a PD subject may include, for example, optimizing sleep, improving sleep quality and/or ameliorating a sleep disorder of any kind.

"Preventing" includes delaying the onset of clinical symptoms, complications, or biochemical indicia of the state, disorder, disease, or condition developing in a subject that may be afflicted with or predisposed to the state, disorder, disease, or condition but does not yet experience or display clinical or subclinical symptoms of the state, disorder, disease, or condition. "Preventing" includes prophylactically treating a state, disorder, disease, or condition in or developing in a subject, including prophylactically treating clinical symptoms, complications, or biochemical indicia of the state, disorder, disease, or condition in or developing in a subject.

The terms "pharmaceutical composition" and "pharmaceutical formulation" as used herein refer to a composition or formulation comprising at least one biologically active compound, for example, levodopa, or a prodrug thereof, such as a levodopa amino acid conjugate, or a pharmaceutically acceptable salt thereof, as disclosed herein, formulated together with one or more pharmaceutically acceptable excipients. It is noted that the terms "formulation" and "composition" are interchangeable unless specifically mentioned otherwise or unless a person skilled in the art would have understood otherwise. The term "pharmaceutically acceptable salt(s)" as used herein refers to salts of acidic or basic groups that may be formed with the conjugates used in the compositions disclosed herein.

"Individual," "patient," or "subject" are used interchangeably and include any animal, including mammals, mice, rats, other rodents, rabbits, dogs, cats, swine, cattle, sheep, horses, or non-human primates, and humans. In some embodiments, the mammal treated in the methods of the invention is a human suffering from neurodegenerative condition, such as Parkinson's disease.

The term "about" as used herein, unless specifically mentioned otherwise, or unless a person skilled in the art would have understood otherwise, is considered to cover a range of ±10% of the listed value(s). It is further noted that any value provided may also be considered to cover a range of ±10% of that value, even without the use of the term "about". This includes the values in the examples section, which may vary according to the utensils and machinery used, the purity of the compounds, etc. The term "liquid" as used herein, unless specifically mentioned otherwise, or unless a person skilled in the art would have understood otherwise, refers to any type of fluid, including gels, aqueous and non-aqueous compositions, and the like.

The terms "continuously" and "substantially continuously" as used herein, unless specifically mentioned otherwise, or unless a person skilled in the art would have understood otherwise, refer to a period of time during which a therapeutic drug (composition or compound) is administered over the entire period of time, optionally with one or more therapeutically effective intermissions. An example intermission may be less than about 24 hours, about 12 hours, about five hours, about three hours, about one hour, about 30 minutes, about 15 minutes, about five minutes or about one minute. The period of time during which a composition is administered may be at least about six hours, about eight hours, about 12 hours, about 15 hours, about 18 hours, about 21 hours, about 24 hours, three days, seven days, two weeks, a month, three months, six months, a year, two years, three years, five years, ten years, etc.

As detailed herein, PD patients may suffer from non-motor symptoms, such as various types of sleep disorders. In certain instances, such non-motor symptoms (e.g., sleep disorders) may be caused by the repetitive or continuous use of levodopa that is administered to treat PD motor symptoms. The present application proposes drug delivery systems and drug delivery methods for ameliorating non-motor symptoms for PD patients, including, but not limited to, PD patients to which levodopa is continuously delivered in order to treat his/her motor symptoms.

Some aspects of the present application may include distinguishing between "apathetic" sleep stages (for example REM sleep stages), the contribution of which to a patient's overall sleep quality is generally minor to non-existent, and "sleep promoting" sleep stages (for example sleep stages 3 and 4), the integrity of which (or lack thereof) has a direct correlation to sleep quality. Namely, the lesser the disruption to the sleep promoting sleep stages, the higher the sleep quality. In general, the drug delivery system described herein can selectively and advantageously deliver a therapeutic agent (e.g., levodopa) to a patient as a function of the patient's actual or anticipated sleep stage. For example, levodopa may be delivered by a drug delivery system to a PD patient at a relatively high flow rate during sleep stages with a minor to non-existant contribution to the overall sleep quality, and whenever the drug delivery system determines (e.g., in real time) from, or based on, a sleep pattern of a PD patient that the PD patient is experiencing a sleep promoting sleep stage (e.g., sleep stage 3), or a sleep promoting sleep stage is anticipated, the drug delivery system may reduce, or start to reduce, the flow rate at which the drug is delivered to the PD patient (which is beneficial in terms of treatment of non-motor symptoms). Delivering levodopa to the PD patient at a relatively high flow rate is beneficial in terms of treatment of motor symptoms, whereas delivering levodopa to the PD patient at a relatively low flow rate is beneficial in terms of treatment of non-motor symptoms.

The drug delivery system may control the drug flow rate by adjusting the values of operational parameters that control operation of a drug dispensing mechanism of the drug delivery system. The drug delivery system may adjust the values of the operational parameters of the drug dispensing mechanism by receiving (e.g., via a communication network), for example from a remote system, updated values for the operational parameters, or by receiving the values locally (e.g., via a local user interface). The drug delivery system may alternatively, or additionally, adjust the values of the operational parameters after having determined the values from sleep data that is associated with the PD patient.

Fig. 1 shows a first configuration of a therapeutic drug delivery system **(100)** for treating a condition associated with a PD subject, for example for optimizing sleep, improving sleep quality and/or ameliorating a sleep disorder in a PD subject according to an example embodiment. In this configuration, therapeutic drug delivery system **100** may include two, separate, subsystems: (1) a drug delivery device ("DDD") **110,** and (2) a sleep monitoring system ("SMS") **120.** The two subsystems **(110,120)** may utilize a same communication protocol, and communicate with one another (e.g., exchange data and commands) via a suitable communication channel **130.** Communication channel **130** may be implemented by a computer communication cable (for example coaxial cable, Ethernet cable, USB cable, etc.), or wirelessly by using any known communication protocol (e.g., BlueTooth, WiFi, GSM (global system for mobile communications), etc.).

DDD **110** may include a controllable drug delivery unit ("DDU") **140,** a controller **150** for controlling **(152)** operation of DDU **140,** a user interface ("UI") **160,** a data storage unit ("DSU") **170,** a communication interface **180,** and a battery **190** (rechargeable or finite) for powering DDD **110.** Communication interface **180** can be configured to, for example, communicate with SMS **120** (as shown in Fig. 1) or, in various embodiments, with any data storage medium (e.g., the cloud or a memory of or server, or a computer hosted by a third party tracker, such as, for example, a FitBit, Whoop Band, Apple Watch, etc.) DDD **110** may be designed to be wearable, or attachable to, a PD subject **112.** DDU **140** may include a therapeutic drug reservoir **142** for storing therein a therapeutic drug, and a controllable dispensing mechanism **144** that is designed (mechanically or electrically, or both mechanically and electrically) to expel therapeutic drug out from therapeutic drug reservoir **142,** in controlled manner **(146),** so as to deliver the expelled therapeutic drug to patient **112,** for example via an infusion catheter **148.**

Controller **150** may be configured to receive sleep data via communication interface **180** or via user interface **160,** and to use the sleep data to calculate values for one or more operational parameters, which controller **150** can use to operate dispensing mechanism **144.** Controller **150** may also be configured to receive (rather than calculate) the values for the operational parameters via communication interface **180** or via user interface **160.**

User interface (UI) **160** enables a user of DDD **110** (for example a physician, a PD patient or a caregiver helping a PD patient) to manually store various types of data (e.g., sleep data corresponding to sleep events that may form a sleep pattern) in DSU **170.** User interface (UI) **160** may also enable the user of DDD **110** to set therapeutically effective values of one or more drug delivery operational parameters (sometimes referred to herein as "operational parameters" for short) for controller **150.** The value(s) that the user may set to the operational parameters may also be stored in DSU **170.** Controller **150** may implement, or use, the parameters' values to control the operation of drug dispensing mechanism **144** according to these value(s).

The values which the user may set to the operational parameters of controller **150** may be selected such that applying them (e.g., by controller **150**) to drug dispensing mechanism **144** would optimize the sleep of the PD subject, improve his/her sleep quality and/or, in general, ameliorate a non-motor symptom from which the PD subject is, or is suspected of, suffering. Example operational parameters, which controller **150** may use to operate drug dispensing mechanism **144,** can be drug flow rate, drug delivery timing (including drug delivery start time and/or drug delivery stop time), increase of drug flow rate per unit of time, decrease of drug flow rate per unit of time, etc., or any combination thereof. Depending on the type of non-motor symptom that is to be ameliorated, different, or additional, operational parameters may be used, and all operational parameters involved may be used solely or collectively (i.e., in conjunction with other operational parameters) to ameliorate one or more non-motor symptoms the PD patient might be experiencing.

User interface (UI) **160** may additionally output an informative feedback signal (visual, audible, and/or haptic) for the user with regard to the stored data and/or with regard to values which have been set by the user to the operational parameters, and/or with regard to the resulting instantaneous (current) drug delivery flow rate and/or drug delivery timing which controller **150** is currently implementing, or which controller **150** is scheduled to implement.

In general, the drug can be delivered to the patient using any known technique. In some embodiments, therapeutic drug reservoir **142** may include a plunger head that is connected to, and is drivable by, a plunger rod. Dispensing mechanism **144** may include a drive unit that may include, for example, an electric motor and a gear unit that is actuated by the electric motor. The gear unit may be configured to engage with the plunger rod of the drug reservoir, and to move the plunger rod, hence the plunger head, linearly. Using the values set to the operational parameters by the user and stored in DSU **170,** controller **150** may control the electric motor such that the electric motor operates the gear unit to move the plunger head at a desired (e.g., at a therapeutically effective) speed (which can be fixed or variable), for example from a clinically desired drug delivery start time until a clinically desired drug delivery stop time. Similar control principles are applicable and contemplated herein for other drug delivery mechanisms.

As described herein (for example above), the user of DDD **110** may use UI **160** to set therapeutically effective value(s) to the operational parameters of controller **150** to enable controller **150** to control the operation of dispensing mechanism **144** according to these value(s). Alternatively, controller **150** may use communication interface **180** to receive sleep data that is based on, or derived from, signals that originate from one or more sleep sensors. A sleep monitoring sensor may be wearable by, or otherwise connected to, the treated PD subject, or it may otherwise (e.g., remotely, such as by an optical system) monitor sleep of the treated PD patient. Controller **150** may use an algorithm to analyze the sleep data it receives via communication interface **180,** and detect or identify, in the received sleep data, one or more sleep events (e.g., REM sleep, sleep stages 1, 2, 3, etc.) that may collectively form a chronologic sleep pattern (CSP) of the involved (treated) PD patient. Controller **150** may determine (e.g., calculate) the values of the operational parameters from, or based on, the CSP. The sleep data that controller **150** may receive via communication interface **180** and the parameters' values determined (e.g., calculated) by controller **150** may also be stored in DSU **170.**

According to a first scenario, controller **150** may use only values that are set to the operational parameters by the user of DDD **110.** According to a second scenario, controller **150** may use only values that controller **150** itself sets to the operational parameters. According to a third scenario, controller **150** may, at certain times, use values that are set to the operational parameters by the user of DDD **110,** but at other times controller **150** may use the values that controller **150** itself set to the operational parameters. In the third scenario, controller **150** may, for example initially, use values that it sets to the operational parameters, but, if desired by the user, the parameters' values set by controller **150** may be overridden (replaced by) values that are set by the user to the operational parameters. In some embodiments, values set (e.g., programmed) by the user for the operational parameters may override the controller-set values. Such override may be performed intermittently, from time to time, for a limited period, etc.

Sleep monitoring system (SMS) **120** may include a controller **122,** a user interface (UI) **124,** a data storage unit (DSU) **126,** a communication interface **128,** and a sensors' interface **132.** Communication interface **128** and communication interface **180** are configured (electrically and software wise) to communicate with one another via communication channel **130.**

Sensors interface **132** may be wired, or be wirelessly connected, to a number ***n*** of sleep sensors (denoted "***Sensor-1***", ***"Sensor-2",..., "Sensor-n"*** in Fig. 1). A sensor of the ***n*** sensors may, in general, be any sensor capable of collecting patient data, e.g., an electrode, a neuronal activity sensor, an EEG sensor, an ECG sensor, an EMG sensor, a polysomnography (PSG) sensor, a sleep sensor, a sleep state sensor, a local field potential sensor, an accelerometer, a wrist watch configured to detect sleep movements, an optical sensor, a camera, etc. Alternative or additional sensors may be connected to sensors interface **132.** The **n** sensors, or some of them, may be physiological sensors that are configured to measure one or more physiological parameters or characteristics of the PD subject. The sensors may provide physiological data (for example physiological data indicative of sleep condition or status) prior, during and/or after a therapeutic drug delivery pattern (DDP) is applied to the PD subject. Some of the sensors may be used to sense spatial movements of the PD subject. A DDP refers to the drug flow rates at which drug is delivered to the PD subject during treatment, and to the timing and duration of each drug flow rate.

The sleep sensors, or some of them, may be wearable by the user (or otherwise be connectable to the user), or they may otherwise be configured to monitor sleep of the user (e.g., a PD patient). The sleep sensors may produce electric signals that represent, or indicate, a sleep stage the user may currently be in, or the current sleep status of the user. Sensors' interface **132** may receive the electric signals that are produced by the **n** sensors, convert the signals to sleep data, and store the resulting sleep data in DSU **126.** Controller **122** may receive a "Request" for sleep data (e.g., sleep data stored in DSU **126**) from DDD **110** via communication channel **130.** Controller **122** may respond to the request by transferring (via communication channel **130**) the requested sleep data to DDD **110,** for further processing by controller **150.** User interface **124** may be used by the user to, for example, select (or deselect) the sensors from which sleep data will be collected (e.g., for storage in DSU **126** ), and/or to manipulate sleep data that is stored in DSU **126,** etc.

Fig. 2 shows a second configuration of a DDD **(200)** for treating a condition associated with a Parkinson's disease (PD) subject, for example for optimizing sleep, improving sleep quality and/or ameliorating a sleep disorder in a PD subject according to another example embodiment. DDD **200** may include a controller **210** for controlling **(220)** operation of DDU **230,** a user interface (UI) **240,** a data storage unit (DSU) **250,** a sensors' interface **260,** and a battery **270** (e.g., rechargeable or finite) for powering DDD **200.** DDD **200** may be designed to be wearable by, or attachable to, a PD subject **212.** DDU **230** may include a therapeutic drug reservoir **232** for storing therein a therapeutic drug, and a controllable dispensing mechanism **234.** Dispensing mechanism **234** may be designed (mechanically or electrically, or both mechanically and electrically) to expel therapeutic drug out from drug reservoir **232,** in a controlled manner **(236),** so as to deliver the expelled therapeutic drug to patient **212,** for example via an infusion set (e.g., catheter) **238.**

Controller **210** may be configured to receive sleep data via sensors' interface **260** or via user interface **240,** or a communication interface included in SMS **280** can be configured to communicate with any data storage medium (e.g., the cloud or a memory of or server or computer hosted by a third party tracker such as, for example, a FitBit, Whoop Band, Apple Watch, etc.) Controller **210** may use the sleep data to determine (e.g., by calculating) the values of one or more operational parameters that controller **210** may use to operate dispensing mechanism **234.** Controller **210** may also be configured to receive (rather than determine) the values for the operational parameters via sensors' interface **260** or via user interface **240.**

DDU **230** may function in the way described herein, for example in connection with DDU **140** of Fig. 1. User interface (UI) **240** may function in the way described herein, for example in connection with UI **160** of Fig. 1. Data storage unit (DSU) **250** may function in the way described herein, for example in connection with DSU **170** and DSU **126** of Fig. 1. Sensors' interface **260** may function in the way described herein, for example in connection with sensors' interface **132** of Fig. 1.

Similar to sensors interface **132,** sensors interface **260** may be wired, or be wirelessly connected, to a number n of sleep sensors (denoted "***Sensor-1***", ***"Sensor-2",..., "Sensor-n"*** in Fig. 1) for monitoring sleep of a user (e.g., a PD patient). The sleep sensors may produce electric signals that represent, or indicate, a user's sleep stage and/or status. Sensors interface **260** may receive the electric signals that are produced by the n sensors, convert the signals to sleep data, and store the resulting sleep data in DSU **250.** A user of DDD **200** may also use user interface **240** to select (or deselect) the sensors for which sleep data will be stored in DSU **250,** and to manipulate the sleep data that is already stored in DSU **250,** etc.

The user of DDD **200** (for example a physician, a PD subject or a caregiver helping a PD subject) may use UI **240** to manually store various types of data (e.g., sleep data corresponding to sleep events that may form a sleep pattern or other health, biological, or physical data related to a user) in DSU **250.** User interface (UI) **240** may also enable the user of DDD **200** to set therapeutically effective values of operational parameters for controller **210.** The value(s) that the user may set to the operational parameters may also be stored in DSU **250.** Controller **210** may implement, or use, the parameters' values to control the operation of drug dispensing mechanism **234** according to these value(s).

The values that the user may set to the operational parameters of controller **210** may be selected such that applying them (by controller **210)** to drug dispensing mechanism **234** would optimize sleep and/or improve sleep quality and/or ameliorate a non-motor symptom from which the PD patient is, or is suspected of, suffering. Example operational parameters, which controller **210** may use to operate drug dispensing mechanism **234,** are described herein, for example in connection with controller **150.**

User interface (UI) **240** may output an informative feedback signal (visual and/or audible) for the user with regard to the stored data and/or with regard to values which have been set by the user to the operational parameters, and/or with regard to the resulting instantaneous (current) drug delivery flow rate and/or drug delivery timing which controller **210** is currently implementing, or which controller **210** is scheduled to implement.

In general, the drug can be delivered to the patient using any known technique. In some embodiments, therapeutic drug reservoir **232** may include a plunger head that is connected to, and is drivable by, a plunger rod. Dispensing mechanism **234** may include a drive unit that may include, for example, an electric motor and a gear unit that is actuated by the electric motor. The gear unit may be configured to engage with the plunger rod of the drug reservoir, and to move the plunger rod, hence the plunger head, linearly. Using the values set to the operational parameters by the user and stored in DSU **250,** controller **210** may control the electric motor such that the electric motor operates the gear unit to move the plunger head at a desired (e.g., at a therapeutically effective) speed corresponding to a desired drug delivery flow rate, which may be fixed or variable, for example according to a therapeutically effective timing.

As described herein (for example above), the user of DDD **200** may use UI **240** to set therapeutically effective value(s) to the operational parameters to enable controller **210** to control the operation of dispensing mechanism **234** according to these value(s). Alternatively, controller **210** may use sleep data that is based on, or derived or originated from, signals that are produced by all, or some of, sleep sensors ***Sensor-1***, ***Sensor-2,..., Sensor-n.*** A sleep sensor may be wearable by, or otherwise connected, to the treated PD patient, or it may otherwise monitor a sleep stage or sleep status of the treated PD patient. Controller **210** may use an algorithm to analyze the sleep data to detect or identify, in the sleep data, one or more sleep events (e.g., sleep stages) that may collectively form a chronologic sleep pattern (CSP) of the involved (treated) PD subject. Controller **210** may determine (e.g., calculate) the values of the operational parameters from, or based on, the CSP. The sleep data and the parameters' values determined (e.g., calculated) by controller **210** may also be stored in DSU **250.**

In a first scenario, controller **210** may use only operational parameter values that are set by the user of DDD **200.** In a second scenario, controller **210** may use only values that controller **210** itself sets to the operational parameters. In a third scenario, controller **210** may, at times, use values that are set to the operational parameters by the user of DDD **200,** but at other times controller **210** may use the values that controller **20** itself sets to the operational parameters. In the third scenario, controller **210** may, for example, initially use values that it sets to the operational parameters, but, if desired by the user, the parameters' values set by controller **210** may be overridden (replaced by) values that are set to the operational parameters by the user. Values set (e.g., programmed) by the user for the operational parameters may override the controller-set values intermittently, for a limited time, or for a remaining sleep period.

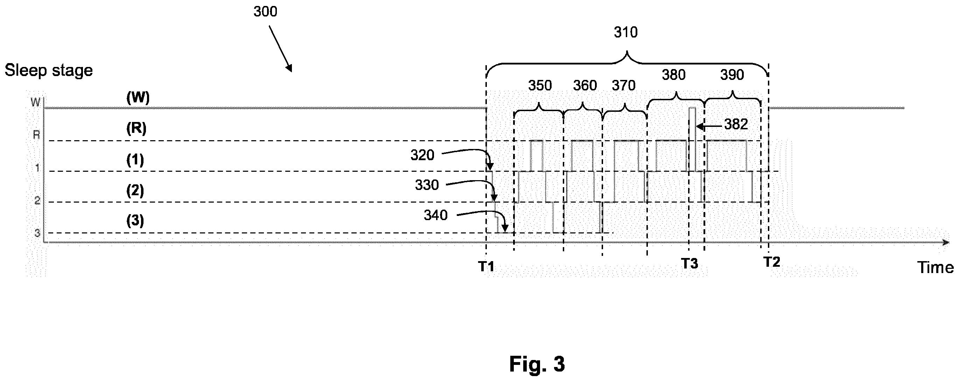

Fig. 3 schematically illustrates an example sleep pattern (hypnogram **300).** A hypnogram represents recordings of, for example, brain wave activity from an electroencephalogram (EEG), and optionally recordings from other sleep monitoring sensors and systems, during sleep. Hypnogram is a form of polysomnography - it is a graph that represents sleep stages as a function of time, for example rapid eye movement sleep (REM) and non-rapid eye movement (NREM) sleep that can be identified during sleep. NREM sleep can be further classified into (include) sleep stage 1, sleep stage 2 and sleep stage 3. Sleep stage 3 may inherently include a fourth sleep stage (sleep stage 4), which is also known as "slow wave sleep" (SWS). Slow wave sleep is the deepest stage of sleep. Each of the three NREM stages, as well as the period of REM sleep and the awake state, can be determined and displayed on a hypnogram.

Hypnogram **300** includes a time axis (the horizontal axis) and a sleep stage axis (the vertical axis). By way of example, the sleep stage axis may include five sleep stages: a "Wake" stage - **(W),** a "REM sleep" stage - **(R),** sleep stage 1 - **(1),** sleep stage 2 - **(2),** and sleep stage 3 - **(3).** Referring to hypnogram **300,** a "total sleep period" **310** starts at time **T1** which, in this example, is a transition time from a "wake" state to sleep stage 1 (shown at **320)** (and then to sleep stage 2, shown at **330,** etc.) and ends at time **T2** which, in this example, is a transition time from sleep stage 1 back to "wake" state. "Total sleep period" **310** is interposed between a preceding awake period and a succeeding awake period.

Normally, a total sleep period includes several sleep cycles. By way of example, hypnogram **300** includes five sleep cycles (n=5) shown, in Fig. 3, as **350, 360, 370 380** and **390.** Some sleep cycles may have 'normal' transitions between the various sleep stages. A sleep cycle may be incomplete or flawed, which means that the sleep cycle might be missing one or more sleep stages, thus impairing the overall sleep quality. Another sleep-depriving problem might be that a person might unintentionally wake up after skipping over one or more sleep stages within a sleep cycle.

Sleep cycles **350** and **360** include all sleep stages both during descending transitions between sleep stages and during ascending transitions between sleep stages. Sleep cycles **350** and **360** include sleep stage 3, which is or includes a deep sleep period. A sleep cycle that includes a slow wave sleep (SWS), which is the deepest stage of sleep, is generally considered to contribute to a generally good quality sleep. Sleep cycle **370** is missing sleep stage 1 during ascending transitions between sleep stages, and sleep stage 3 during descending transitions between sleep stages. Therefore, sleep cycle **370** disrupts the overall sleep quality. Sleep cycle **380** is missing sleep stage 3 during both descending and ascending transitions between sleep stages. Sleep cycle **380** also includes an abnormal (an unintentional) transition **382** (at time **T3)** between sleep stage 1 and "wake up" stage (W). Therefore, sleep cycle **380** also disrupts the overall sleep quality. Sleep cycle **390** is also missing sleep stage 3 during both descending and ascending transitions between sleep stages. Therefore, sleep cycle **390** also disrupts the overall sleep quality.

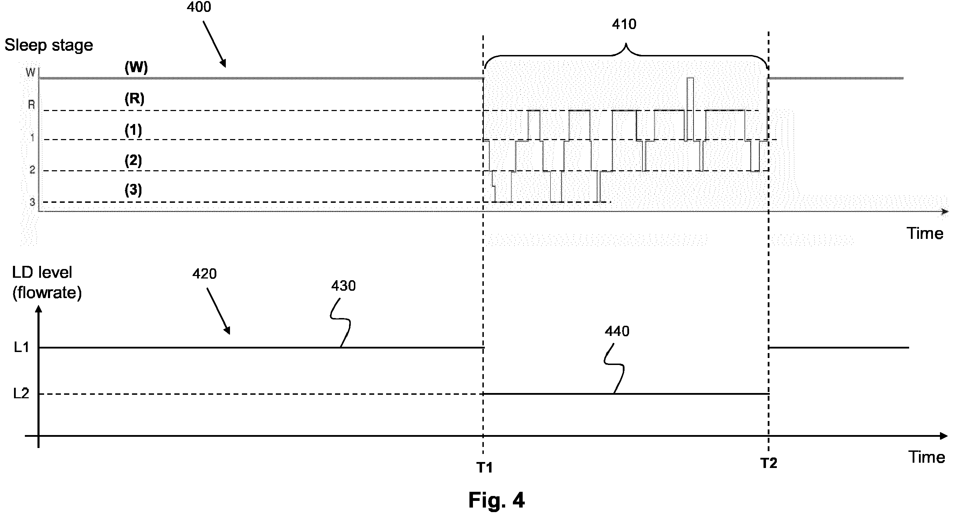

Fig. 4 schematically illustrates a drug delivery control scheme for controlling flow rate of a therapeutic drug delivered to a PD subject to treat a condition associated with a PD subject. A drug delivery control scheme is determined, or defined, by the values that are set to the operational parameters used to operate the drug delivery unit (e.g., DDU **140,** DDU **230).** A particular drug delivery control scheme defines a particular pattern which is used to deliver the drug to a PD subject. The values that are set to the operational parameters, therefore, indirectly define a drug delivery pattern (DDP), so drug delivery control scheme (DDCS) and DDP are interrelated. Treating a condition may include, for example, optimizing sleep, improving sleep quality and/or ameliorating a sleep disorder, to name a few. Fig. 4 shows an example hypnogram **400** embodying an example sleep pattern **410** of a PD subject, and a 'toggle' ("On"/"Off") kind of therapeutic drug flow rate control scheme **420** as a function of the sleep pattern. Briefly, "on"/"off" drug flow rate control scheme **420** includes initially delivering the therapeutic drug to the PD subject at a high flow rate **(430)** when the PD subject is awake, and at a lower flow rate **(440)** when the PD subject is asleep (e.g., between time **T1** and awake time **T2).**

During daytime, for example, PD subjects are or can be treated by continuously delivering a therapeutic drug (typically levodopa or levodopa-carbidopa based formulation) to the PD subject in order to treat motor symptoms of the PD subject. To make the motor treatment effective, the therapeutic drug is often delivered to PD subjects at a relatively high flow rate (e.g., at flow rate level **L1**), which is a flow rate level shown at **430** in Fig. 4. The value of **L1** may be selected, for example, from the range 0.50ml/h - 0.80ml/h, for example the value of **L1** may be 0.64ml/h. The value of **L2** may be selected, for example, from the range 0.05ml/h - 0.10ml/h, for example the value of **L2** may be 0.08ml/h.

When a PD subject falls asleep, motor symptoms may be less severe compared to the manifestation of motor symptoms during daytime when the PD subject is awake and needs help controlling his/her involuntary movements. In addition, when the PD subject goes to bed or falls asleep, s/he may be required to decrease the night drug delivery flow rate to maintain the total night drug dose low in order to avoid drug overdose during the night. In some PD subjects, high drug dose during the night causes sleep disorders. Therefore, it may be beneficial to deliver the therapeutic drug at a lower flow rate during the nighttime, in particular when a sleep monitoring system indicates that the PD subject has just fallen asleep. For some PD subjects it may be therapeutically and sleep-wise beneficial to use a low drug delivery flow rate for the entire total sleep period (e.g., during period **410),** while for other PD subjects it might be therapeutically and sleep-wise beneficial to use a low drug delivery flow rate only during certain time periods within the total sleep period **410.** Other PD subjects might require that, for an efficient treatment to be rendered, the drug delivery flow rate be changed according to another scheme. In other words, a drug delivery flow rate may be changed according to a drug delivery control scheme that is tailored to the specific treatment needs of a specific PD subject, both in terms of treatment of motor symptoms and treatment of non-motor symptoms (e.g., sleep disorders).

It is noted that while two flow rates (e.g., **L1** and **L2)** are contemplated in Figures 4, 5 and 6, further embodiments of the invention are directed to the implementation of any number of flow rates (e.g., **L1, L2,..., Lm;** m=1, 2, 3, ...,) as desired, or required, or beneficial to the PD subject. For example, there may be a medium rate between **L1** and **L2,** implemented at certain times, e.g., replacing, or partially replacing, any of the **L1** and/or **L2** rates depicted in Figures 4, 5 and/or 6. There may further be a rate lower than **L2,** or higher than **LI,** implemented at any appropriate time. Further, there may be times where the flow rate is 0.00ml/h, such that no or essentially no drug is administered for a certain length of time, wherein the length of time may be determined as detailed herein, by data (e.g., sleep data, movement data, etc.) received from sensors, the well-being of the PD patient, as noted by a physician, a caregiver, or the PD patient him/herself.

It is further noted that the administration of varying flow rates, as detailed herein, may include both night rates and day rates; however, during the day, sleep episodes may be accounted for as well, such that when the PD subject sleeps or rests during the day, the drug delivery flow rate may be altered, as detailed herein.

In addition, it is noted that all references to a PD subject "falling asleep", and the like, may also refer to a PD subject "resting", i.e., having low activity during which time the change in flow rate, as detailed herein, may be appropriate as well. Similarly, references to "waking", and the like, may include "becoming more active", such that a change in flow rate, as detailed herein, may be appropriate. Further, references to "daytime" and "nighttime", and the like, are considered to include references to "active times" or "highly active times" and "non-active times" or "low active times", respectively.

Referring again to Fig. 4, which illustrates an example drug delivery control scheme, at time **T1** the PD subject falls asleep (the subject's sleep onset may be sensed or detected by a sleep monitoring system). Given the explanation above about the drug delivery flow rate recommended during sleep, at time **T1** the drug delivery flow rate is decreased from level **L1** to level **L2** (level **"L2"** is shown at **440; L1>L2).** At time **T2** the PD subject wakes up (per indication given by the sleep monitoring system) and, given the explanation above, with regard to the drug delivery flow rate recommended during wake time, at time **T2** the high drug delivery flow rate is resumed, by increasing it from flow rate level **L2** back to flow rate level **L1**.

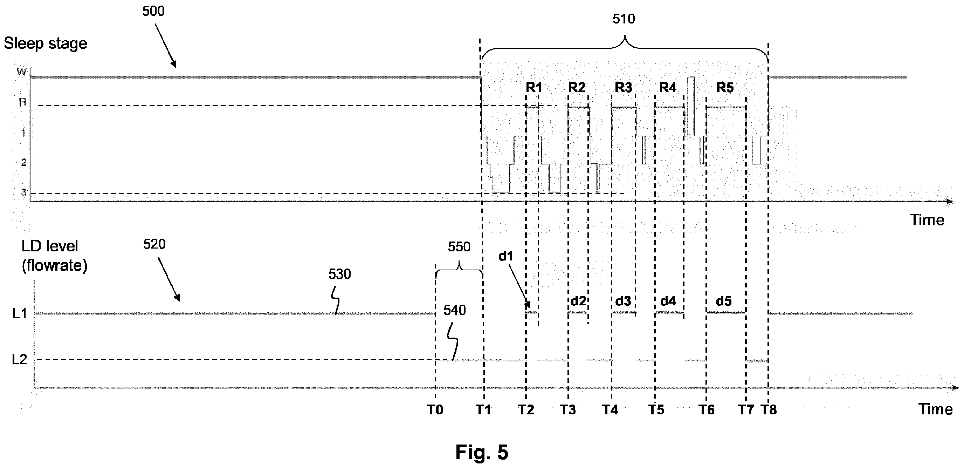

Fig. 5 schematically illustrates a control scheme for controlling flow rate of a therapeutic drug delivered to a PD subject to treat a condition associated with a Parkinson's disease (PD) subject, for example optimizing sleep, improving sleep quality and/or ameliorating a sleep disorder, according to another example control scheme. In this example, the drug delivery flow rate control scheme is targeted at (for example adapted to or derived from) the REM sleep events identified in the sleep pattern. Another reason for targeting the drug delivery flow rate control scheme at the REM sleep events is ensuring that sleep stage 3 (the deepest sleep stage) is not disrupted. Disrupting sleep stage 3 results in poor sleep quality which is typically undesirable. Briefly, drug flow rate (drug delivery) control scheme **520** discloses delivering of therapeutic drug to the PD subject at a relatively high flow rate during certain sleep events which, in the example of Fig. 5, are REM sleep events **R1**, **R2,..., R5.**

Fig. 5 shows an example hypnogram **500** embodying an example sleep pattern **510** of a PD subject, and an example therapeutic drug flow rate control scheme (graph **520)** as a function of sleep pattern **510.** By way of example, sleep pattern **510** starts at time **T1** and ends at time **T8,** and includes five REM sleep periods: REM sleep period **R1** that starts at time **T2,** REM sleep period **R2** that starts at time **T3,** REM sleep period **R3** that starts at time **T4,** REM sleep period **R4** that starts at time **T5,** and REM sleep period **R5** that starts at time **T6.** Each REM sleep period represents, or is associated with, a different sleep cycle.

As explained herein, the drug delivery flow rate is relatively high, i.e., at level **L1** (shown at **530** in Fig. 5, **L1**>**L2**) during the daytime (when the PD subject is awake) in order to efficiently treat motor symptoms. Assume in this example (e.g., based on history sleep data related to the specific PD subject) that the sleep onset time of the PD subject is usually at, or near, time **T1**. At time **T0**, which precedes time **T1**, the PD subject is still awake but, nevertheless (i.e., according to the example drug delivery flow rate control scheme of Fig. 5), the drug delivery flow rate changes from high drug flow rate level **L1** (shown at **530)** to a lower drug flow rate level **L2** (shown at **540)** in preparation for entering sleep mode in which lower total drug dose would be beneficial in terms of overall sleep quality. The time difference **550 (Td)** preceding sleep onset time **T1** (i.e., time difference **Td**=**T1**-**T0**) may be determined based, for example, on the severity of the motor symptom(s) and/or non-motor symptom(s) (e.g., sleep disorder(s)) experienced by the PD subject. For example, the more severe a motor symptom of a PD subject is, the smaller the value of **Td** can be in order to maintain the high flow rate of the therapeutic drug (which is useful in treating motor symptoms) for as long as possible as the PD subject is nearing, or entering, sleep mode. In an opposite example, if the motor symptom(s) experienced by the PD subject is moderate, the time difference **(Td)** may be relatively long to, thus, reduce the overall drug dose expected during sleep to an amount that, on the one hand, would help avoiding, or suppressing, non-motor symptoms (e.g., sleep disorder(s)) while, on the other hand, would still be effective in managing the motor symptom(s) of the PD subject.

Sleep studies suggest that sleep stage 3 is the deepest sleep period during sleep, and that deterioration in sleep quality and/or sleep quantity is correlated with sleep disruptions that occur during sleep stage 3. Generally, the greater a disruption to sleep stage 3, the greater the deterioration in the overall sleep quality. Sleep studies also suggest that excessive delivery of drug (e.g., levodopa) dose during sleep may disrupt sleep stage 3. Therefore, it would be beneficial to use low drug delivery flow rate during sleep in general, and during the deep sleep stage (sleep stage 3 or sleep stage 4) in particular, to optimize sleep quality.

Since the REM sleep stage has lower effect on sleep quality than sleep stage 3, drug delivery can be done at relatively high flow rate (e.g., at rate level **L1** in Fig. 5) during the REM sleep stages **R1**, **R2,..., R5** without significantly compromising sleep quality. Accordingly, the drug delivery flow rate can be maintained at the low level **(L2,** at **540)** from time **T0** until **T2** (the time at which the first REM sleep (**R1**) event commences), and changed to the high level (**L1**) at time **T2** (the time at which the first REM sleep (**R1**) event commences) for a time duration **d1** that corresponds to (e.g., overlaps, at least partially, or coincides with) the REM sleep event **R1**. When REM sleep event **R1** ends (or shortly before or after it ends), the drug delivery flow rate is decreased to low level **L2** until time **T3.** The drug delivery flow rate resumes the high level (**L1**) at time **T3** (the time at which the second REM sleep (**R2**) event commences) for a time duration **d2** that corresponds to (e.g., overlaps, at least partially, or coincides with) the REM sleep event **R2.** The same drug delivery alternating flow rate process may also apply to each consecutive REM sleep event, for example to REM sleep events **R3, R4,** and **R5.** Fig. 5, therefore, demonstrates a pulsatile type of drug flow rate control.

The drug delivery flow rate level in each REM sleep event may change as shown in Fig. 5 and described above; i.e., the drug flow rate can be increased from low level **L2** to the same high level (e.g., to level **L1**) for all REM sleep events, but, in other scenarios, the drug flow rate may be increased to other high flow rate levels, some of which may be lower or higher than level **L1**. By way of example, the drug delivery flow rate value during REM sleep event **R1** may be **L1-delta_1,** during REM sleep event **R2** it may be **L1**+**delta_2,** during REM sleep event **R3** it may be **L1-delta_3,** etc. ("**delta_1**", "**delta_2**", "**delta_3**", etc., may be, for example, a percentage of 'basic' flow rate level **LI,** for example about 5%, 3%, 2%,..., etc., of the flow rate level **LI,** respectively.)

The drug delivery flow rate level between REM sleep events may change as shown in Fig. 5 and described above; e.g., the drug flow rate can be decreased from high level **L1** to the same low level (e.g., to level **L2)** following each REM sleep event, but, in other scenarios, the drug flow rate may be decreased to other low flow rate levels, some of which may be lower or higher than low level **L2.** By way of example, the drug delivery flow rate value for the time period following REM sleep event **R1** (i.e., the time space between REM sleep event **R1** and REM sleep event **R2)** may be **L2-delta_1**; the drug delivery flow rate value for the time period following REM sleep event **R2** (i.e., the time space between REM sleep event **R2** and REM sleep event **R3)** may be **L2+delta_2**; the drug delivery flow rate value for the time period following REM sleep event **R3** (i.e., the time space between REM sleep event **R3** and REM sleep event **R4)** may be **L3-delta_3**; etc. ("**delta_1**", "**delta_2**", "**delta_3**",..., etc., may be, for example, a percentage (e.g., about 6%, 4%, 1%, etc., respectively) of 'basic' flow rate level **L2.)** In some scenarios, the drug delivery flow rate can be changed according to a 'combined' scheme; namely, both basic levels **L1** and **L2** may be modified by using the modifier "**delta_i**" (where i=1, 2, 3, ...).

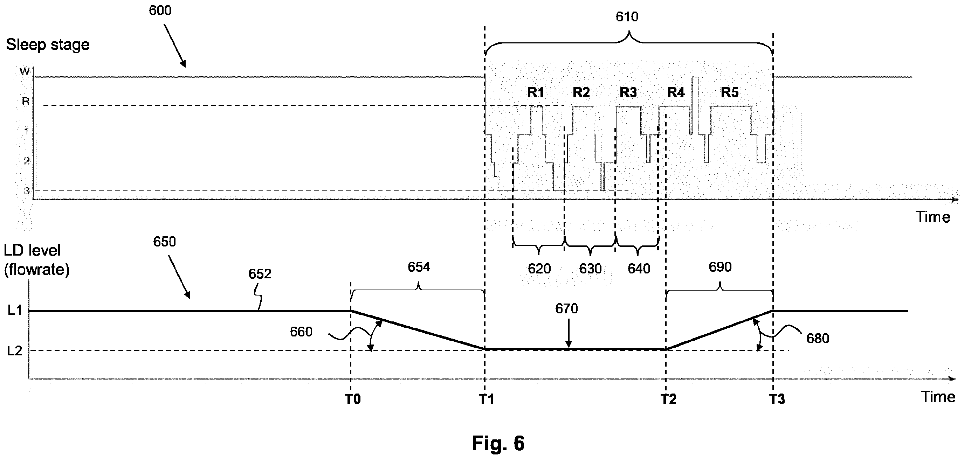

Fig. 6 schematically illustrates a drug delivery control scheme for controlling flow rate of a therapeutic drug delivered to a PD subject to treat a condition associated with a PD subject, for example, optimizing sleep, improving sleep quality and/or ameliorating a sleep disorder in accordance with another example embodiment. Briefly, drug flow rate control scheme **650** includes gradually lowering (for example from a relatively high daytime drug delivery flow rate) the drug delivering flow rate, between time **T0** and time **T1**, prior to (e.g., in anticipation for) the PD subject entering a sleep mode; then maintaining the low drug delivery flow rate during sleep (between time **T1** to time **T2),** and, finally, gradually increasing (for example back to the daytime drug delivery flow rate) the drug delivering flow rate, between time **T2** and time **T3,** in anticipation to the PD subject awaking.

Fig. 6 shows an hypnogram **600** embodying a sleep pattern **610** of a PD subject, and a therapeutic drug flow rate control graph **650** as a function of the sleep pattern. In this example, the drug delivery flow rate control scheme is targeted at (depends or based on or derived from) a predetermined number "n" of sleep cycles of the subject's sleep pattern. By way of example, sleep pattern **610** starts at time **T1** (sleep onset time) and ends at time **T3** (wakeup time), and includes five REM sleep events: REM sleep event **R1**, REM sleep event **R2,** REM sleep event **R3,** REM sleep event **R4,** and REM sleep event **R5.** Each REM sleep event is associated with (therefore can represent) a different sleep cycle. By way of example, three sleep cycles are shown as **620, 630** and **640.**

In this example, before and up until time **T0** the PD subject is awake. During that time, the drug delivery flow rate is at high level **(L2;** shown at **652)** in order to ameliorate motor symptoms of the subject. At time **T0**, which precedes sleep onset time **T1**, the PD subject is still awake but according to the example drug delivery flow rate control scheme of Fig. 6, the drug delivery flow rate decreases gradually (e.g., linearly, monotonically, stepwise, etc., as shown at **654),** for example at a decreasing angle **660,** from high drug flow rate level **L1** to a lower drug flow rate level **L2,** in preparation (in anticipation) for entering sleep mode with reduced drug dose, which would be beneficial in terms of overall sleep quality. The time difference **Td** (time space **654)** preceding sleep onset time **T1** (i.e., **Td**=**T1**-**T0**) and the decreasing angle **660** of the descending flow rate between **T0** and **T1** may be determined based on, for example, the severity of the motor symptom(s) and/or non-motor symptom(s) (e.g., sleep disorder(s)) experienced by the PD subject.

According to the drug delivery flow rate control scheme of Fig. 6, the drug delivery flow rate can be maintained at low level, for example at flow rate level **L2** (shown at **670)** and for a number n of consecutive sleep cycles. By way of example, Fig. 6 shows three (n=3) consecutive sleep cycles (sleep cycles **620, 630** and **640)** during which the drug delivery flow rate is maintained at low level **L2.** Keeping the drug delivery flow rate at low level **(L2)** during the three consecutive sleep cycles may be beneficial because one or more of sleep cycles **620, 630** and **640** may include sleep stage 3 (the deepest sleep stage), and keeping the drug flow rate at low level during sleep stage(s) 3 would likely to prevent the deep sleep stage from being disrupted, thereby increasing the chance of the subject having normal (undisrupted), or close to normal, sleep.

Time **T2** roughly indicates the completion of the three sleep cycles **620, 630** and **640.** Assuming in this example (based, for example, on historical sleep data) that a sleep pattern of the involved PD subject includes a total of, for example, five sleep cycles (additional two sleep cycles are denoted in Fig. 6 as **"R4"** and **"R5"),** it may be decided (e.g., by DDD **100** of Fig. 1, or by DDD 200 Fig. 2) that after completion of the three sleep cycles **620, 630** and **640** (60% of the total number of five sleep cycles) the PD subject is about to wake up. Therefore, at time **T2,** the drug delivery flow rate is gradually (e.g., linearly, monotonically, stepwise, etc.) increased, for example at an increasing angle **680,** until the drug delivery flow rate reaches high flow rate level **L1** at time **T3,** which is the time the PD subject is, or expected to be, fully awake. The time immediately following the n sleep cycles, i.e., the time between times **T2** and **T3,** may be regarded as a "pre-awakening period". Gradually increasing the drug delivery flow rate between time **T2** and time **T3** (e.g., while the PD subject is still asleep during the pre-awakening period) gradually elevates the level of drug in the PD subject in preparation for his/her awakening. Such preparation helps the PD subject to get out of bed, get dressed, and, in general, do whatever s/he normally does after waking up.

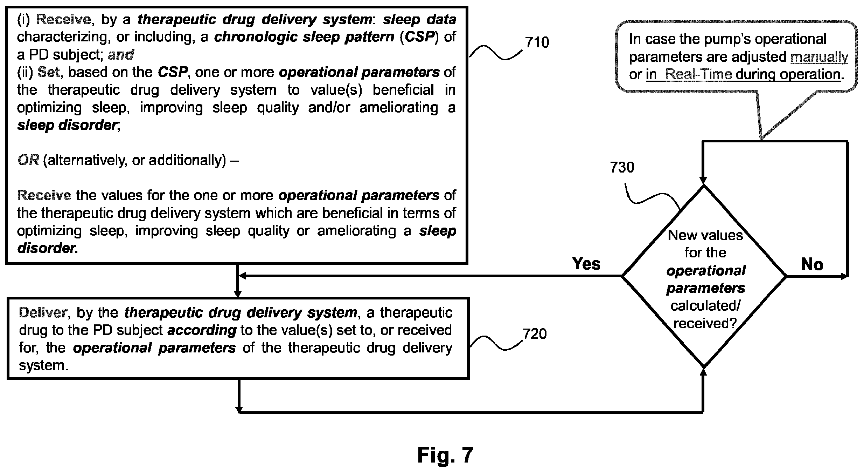

Fig. 7 shows a method of operating a therapeutic drug delivery system for treating a condition associated with a Parkinson's disease (PD) subject, for example for optimizing sleep, improving sleep quality and/or ameliorating a sleep disorder according to an example embodiment. Fig. 7 will be described in association with Fig. 1.

At step 710, therapeutic drug delivery system **100** may, in some embodiments, receive sleep data characterizing, or including, a chronologic sleep pattern (CSP) of a PD subject, and set, based on the CSP, one or more operational parameters of the therapeutic drug delivery system to value(s) which are beneficial in terms of optimizing sleep, improving sleep quality in general and/or ameliorating sleep dysfunction. Alternatively (e.g., in other embodiments), therapeutic drug delivery system **100** may receive the values for the one or more operational parameters of the therapeutic drug delivery system from an external system.

At step 720, therapeutic drug delivery system **100** may deliver a therapeutic drug to the PD subject according to the value(s) set to, or received for, the operational parameters of the therapeutic drug delivery system. The values set to, or received for, the operational parameters of therapeutic drug delivery system **100** may be used throughout the sleep period, or they may be adjusted from time to time, or in real time (by using newly received, or newly determined, operational parameter values). The adjustments, either in real time or from time to time, e.g., at predesignated time intervals, or when certain objective or subjective indications or parameters suggest adjustments would be beneficial, may be performed automatically, e.g., by, or based on, input from the system, or, for example, manually, e.g., by the PD subject, a physician or a caregiver.

At step 730, therapeutic drug delivery system **100** may check whether new values for the operational parameters have been received or need to be determined. If such values are neither received nor needed to be determined (this is shown as **"No"** at step 730), therapeutic drug delivery system **100** may continue to use the values that were received or determined last. However, if therapeutic drug delivery system **100** receives new values for the operational parameters, or it needs to recalculate the values (shown as **"Yes"** at step 730), therapeutic drug delivery system **100** delivers the therapeutic drug to the PD subject according to the new value(s) that were set to, or received for, the operational parameters of, therapeutic drug delivery system **100.**

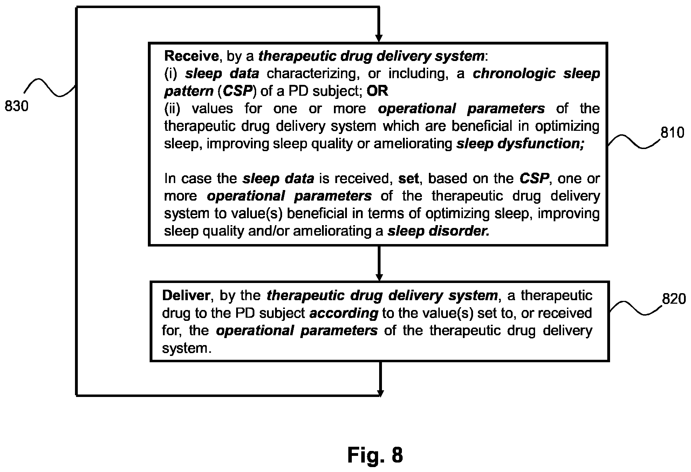

Fig. 8 shows a method of operating a therapeutic drug delivery device for treating a condition associated with a PD subject, for example for optimizing sleep, improving sleep quality and/or ameliorating a sleep disorder optimize sleep, improve sleep quality and/or ameliorate a sleep disorder according to another example embodiment. Fig. 8 will be described in association with Fig. 2.

At step 810, therapeutic DDD **200** may receive either sleep data characterizing, or including, a chronologic sleep pattern (CSP) of a PD subject, or values for one or more operational parameters of therapeutic DDD **200,** which are beneficial in terms of optimizing sleep, improving sleep quality or ameliorating sleep dysfunction. If therapeutic DDD **200** receives the sleep data, therapeutic DDD **200** (e.g., controller **210)** may calculate and set, based on the CSP, one or more operational parameters of the therapeutic drug delivery system to value(s) that are beneficial, for example, in terms of optimizing sleep, improving sleep quality and/or ameliorating sleep dysfunction.

At step 820, therapeutic DDD **200** may deliver (for example by controller **210** controlling operation of dispensing mechanism **234)** a therapeutic drug from drug reservoir **232** to the PD subject according to the values set by controller **210** to, or received by controller **210,** for the operational parameters of therapeutic drug delivery system **210.**

Control loop 830 signifies various options to adjust (e.g., by controller **210)** the values of the operational parameters that controller **210** may use to control drug delivery unit (DDU) **230** to deliver the therapeutic drug to the PD subject. For example, the values of the operational parameters may be adjusted (e.g., preprogrammed) manually by the PD subject, a caregiver and/or a physician (e.g., via user interface **240)** at any time (e.g., before bedtime, when the subject awakens during the night, etc.). Alternatively, or additionally, the values of the operational parameters may be adjusted automatically (e.g., by controller **210)** in real time based on real time sleep data that controller **210** may receive (and use to calculate or determine the new values of the operational parameters), or based on new operational parameters values that controller **210** may receive.

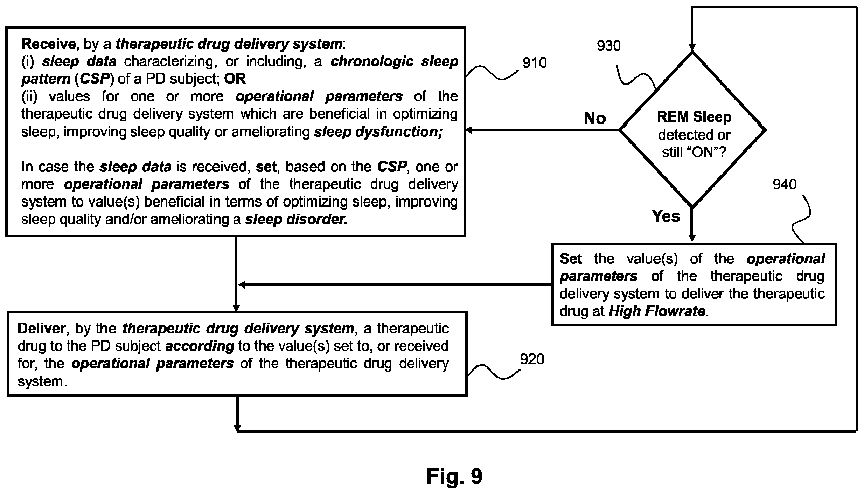

Fig. 9 shows a method of operating a therapeutic drug delivery device for treating a condition associated with a Parkinson's disease (PD) subject, for example for optimizing sleep, improving sleep quality and/or ameliorating a sleep disorder optimize sleep, improve sleep quality and/or ameliorate a sleep disorder according to yet another example embodiment. Fig. 9 will be described in association with Fig. 2 and Fig. 5.

As described in connection with Fig. 5 (for example), drug delivery flow rate during REM sleep events can be high because, as explained in the description of Fig. 5, disruptions during REM sleep events usually do not have much, or substantial, bearing on sleep quality. Accordingly, the drug delivery control scheme of Fig. 9 is based on detection of REM sleep events within a sleep pattern of a PD subject, and setting the values of the involved operational parameters to values corresponding to high drug flow rate during each detected REM sleep event.

At step 910, therapeutic DDD **200** may receive either sleep data characterizing, or including, a chronologic sleep pattern (CSP) of a PD subject, or values for one or more operational parameters of therapeutic DDD **200,** which are beneficial in terms of optimizing sleep, improving sleep quality or ameliorating sleep dysfunction. If therapeutic DDD **200** receives the sleep data, therapeutic DDD **200** (e.g., controller **210)** may calculate and set, based on the CSP, one or more operational parameters of the therapeutic drug delivery system to value(s) that are beneficial in terms of optimizing sleep, improving sleep quality and/or ameliorating sleep dysfunction.

At step 920, therapeutic DDD **200** may deliver (for example by controller **210** controlling operation of dispensing mechanism **234)** a therapeutic drug from drug reservoir **232** to the PD subject according to the value(s) set by controller **210** to, or received by controller **210** for, the operational parameters for controlling the drug delivery flow rate.

At step 930 controller **210** checks (for example based on real time sleep data that controller **210** may receive), whether a new, or additional, REM sleep event has commenced. If controller **210** determines at step 930 that a new, or another, REM sleep event has commenced (the condition being shown as **"Yes"** at step 930), controller **210** may, at step 940, set the operational parameters to values suitable for controlling drug delivery unit (DDU) **230** to deliver the therapeutic drug to the PD subject at high flow rate. Controller **210** may maintain the drug delivery at high flow rate for as long as the current REM sleep event in "on" (this condition is being checked at step 930). However, if controller **210** determines, at step 930, that a current REM sleep event has ended (the condition being shown as **"No"** at step 930), controller **210** may reuse, at step 910, the operational parameters values that were last received or determined by controller **210,** and use them, at step 920, to deliver the therapeutic drug from drug reservoir **232** to the PD subject at a drug delivery flow rate corresponding to these values.

Controller **210** may revisit step 930 to see if there are additional REM sleep events, and manage each such REM sleep event in the way described above; namely, set the value(s) of the operational parameters of the therapeutic drug delivery system to deliver the therapeutic drug at high flow rate during, and for the duration of, the REM sleep event. Controller **210** may terminate the drug delivery process when the PD subject wakes up.

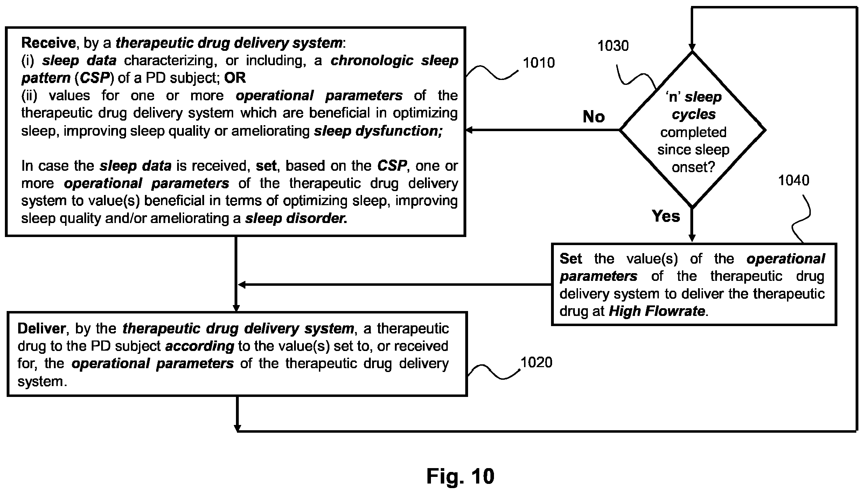

Fig. 10 shows a method of operating a therapeutic drug delivery device for treating a condition associated with a Parkinson's disease (PD) subject, for example optimizing sleep, improving sleep quality and/or ameliorating a sleep disorder for optimizing sleep, improving sleep quality and/or ameliorating a sleep disorder according to yet another example embodiment. Fig. 10 will be described in association with Fig. 2 and Fig. 6.

As described in connection with Fig. 6 (for example), a drug delivery flow rate can beneficially be low during the first n consecutive sleep cycles in a sleep pattern (e.g., sleep cycles **620, 630** and **640** in Fig. 6; i.e., n=3 in Fig. 6) out of a total number of sleep cycles that is greater than n. The reason for this is that low drug dose during that time would contribute to, or promote, sleep quality. By way of example, the n sleep cycles can be 50% of the total number of sleep cycles during the night, or 70% of the total number of sleep cycles during the night, or another percentage of the total number of sleep cycles within the related sleep pattern. During the sleep cycles that follow the n consecutive sleep cycles the drug delivery flow rate may beneficially be increased gradually (e.g., linearly, monotonically, stepwise), as shown in Fig. 6 at **690,** in order to prepare the PD subject for awakening in the morning.

At step 1010, therapeutic DDD **200** may receive either sleep data characterizing, or including, a chronologic sleep pattern (CSP) of a PD subject, or values for one or more operational parameters of therapeutic DDD **200,** which are beneficial in terms of optimizing sleep, improving sleep quality or ameliorating sleep dysfunction. If therapeutic DDD **200** receives sleep data, controller **210** may use the CSP to calculate drug delivery values that are beneficial in terms of, for example, optimizing sleep, improving sleep quality and/or ameliorating sleep disorder, and set one or more operational parameters of DDD **200** to these values.

At step 1020, therapeutic DDD **200** may deliver (for example by controller **210** controlling operation of dispensing mechanism **234)** a therapeutic drug from drug reservoir **232** to the PD subject according to the values set by controller **210** to, or according to the values received by controller **210** for, the operational parameters for controlling the drug delivery flow rate. Referring to Fig. 6, the drug delivery flow rate controlling scheme that controller **210** may implement at this stage may include (in this example) linearly, or gradually, decreasing the drug delivery flow rate in the 'pre-sleeping' period (as shown at **654** in Fig. 6), which is a short time period preceding the sleep onset time; in the example of Fig. 6 it is the time space between time **T0** and sleep onset time **T1**.

At step 1030 controller **210** may check (for example based on historic sleep data and/or based on real time sleep data that controller **210** may receive) the number of consecutive sleep cycles that passed from the sleep onset time, for example from sleep onset time **T1** (see Fig. 6). If controller **210** determines, at step 1030, that the number of sleep cycle that passed from the sleep onset time (e.g., from sleep onset time **T1**) is 'n' (the condition being shown as **"Yes"** at step 1030), controller **210** may set, at step 1040, the operational parameters to values that are suitable for controlling drug delivery unit (DDU) **230** to deliver therapeutic drug to the PD subject at a gradually (e.g., linearly, monotonically, stepwise, etc.) increasing flow rate (shown at **690** in Fig. 6 as angle **680),** in anticipation for the PD subject awakening, for example at time **T3** in Fig. 6. Then, at step 1020, controller **210** delivers the therapeutic drug to the PD subject according to the value(s) it has set to the operational parameters at step 1040; namely, controller 210 starts to deliver drug to the PD subject at a gradually increasing flow rate. However, if controller **210** determines, at step 1030, that the number of consecutive sleep cycles is still lower than 'n' (the condition being shown as **"No"** at step 1030), controller **210** may revisit step 1010 and, for example, may maintain the drug delivery flow rate at low level (e.g., as shown at **670** in Fig. 6) in order to avoid disrupting the subject's sleep. Controller **210** may wait until 'n' consecutive sleep cycles pass before it starts to gradually (e.g., linearly, stepwise, etc.) increase the drug delivery flow rate towards awakening of the PD subject.

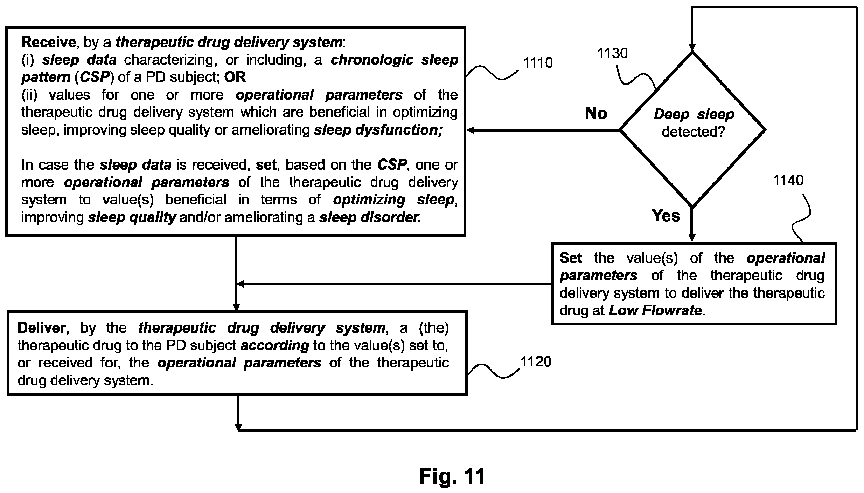

Fig. 11 shows a method of operating a therapeutic drug delivery device for treating a condition associated with a Parkinson's disease (PD) subject, for example for optimizing sleep, improving sleep quality and/or ameliorating a sleep disorder for optimizing sleep, improving sleep quality and/or ameliorating a sleep disorder according to an example embodiment. Fig. 11 will be described in association with Fig. 2. As described herein, it may be beneficial to deliver drug to the PD subject at low flow rate during sleep stage 3 (the deepest sleep stage in a sleep cycle) to avoid sleep disruption during this critical sleep stage.

At step 1110, DDD **200** may receive (for example via user interface **240)** either sleep data characterizing, or including, a chronologic sleep pattern (CSP) of a PD subject, or values for the one or more operational parameters of DDD **200** which may be beneficial in terms of optimizing sleep, improving sleep quality and/or ameliorating a sleep disorder. If DDD **200** receives the sleep data, controller **210** may use the CSP to calculate and to set the one or more operational parameters of DDD **200** to values that may, for example, optimize sleep, improve sleep quality and/or ameliorate a sleep disorder in the PD subject. At step 1120, DDD **200** may deliver (for example by controller **210** controlling operation of dispensing mechanism **234)** a therapeutic drug from drug reservoir **232** to the PD subject according to the values that controller **210** sets to, or receive for, the operational parameters for controlling the drug delivery flow rate.

At step 1130 controller **210** analyzes the sleep data it receives in real time (e.g., via sensors interface **260),** or sleep data it receives via user interface **240,** in order to determine whether the PD subject is currently in a sleep stage 3 (deep sleep stage). If controller **210** determines, at step 1130, that the PD subject is currently in a sleep stage 3 (the condition being shown as **"Yes"** at step 1130), controller **210** may set, at step 1140, the operational parameters to values that are suitable for controlling drug delivery unit (DDU) **230** to deliver therapeutic drug to the PD subject at a low flow rate. Then, at step 1120, controller **210** delivers the therapeutic drug to the PD subject according to the value(s) it has set to the operational parameters at step1140. However, if controller **210** determines, at step 1130, that the sleep stage the PD user is currently in is not sleep stage 3 (the condition being shown as **"No"** at step 1130), controller **210** returns to step 1110 to determine the next values for the operational parameters that controller **210** may use (at step 1120) to deliver the therapeutic drug to the PD subject during the other sleep stages (i.e., in the non-sleep 3 stages).

Fig. 12 shows a third example configuration of a therapeutic drug delivery system ("DDS") **1200** for treating a condition associated with a Parkinson's disease (PD) subject, for example for optimizing sleep, improving sleep quality and/or ameliorating a sleep disorder for optimizing sleep, improving sleep quality and/or ameliorating a sleep disorder according to yet another example embodiment. DDS **1200** may include a DDD **1210** and an operational parameters calculation system ("OPCS") **1220.**

Referring again to Figs. 1 and 2, drug delivery system **100** and drug delivery system **200** are local systems; namely, all parts of system **100** (e.g., the system's drug delivery device and sleep monitoring system) are typically in a same physical location, which is in the premises of the PD subject. Similarly, all parts of DDD **200** are typically located in a same physical location, which is in the premises of the PD subject. In contrast, DDS **1200** proposes means by which calculation of the values of the drug delivery operational parameters may be performed anywhere; that is, locally (e.g., in the premises of the PD subject) and/or remotely (e.g., by or in a remote system), even though the pertinent sleep data is produced (e.g., by a SMS or other physiological tracker worn by the PD subject, e.g., a Fitbit, Whoop Band, Apple Watch, etc.), or otherwise provided (e.g., via a user interface) in the premises of the PD subject. An example communication system and method that facilitate such network-based drug delivery scheme is described below.

DDD **1210** may include a controller **1212,** a sleep monitoring system (SMS) **1214** for receiving sleep signals from one or more sleep sensors and, optionally, for generating sleep data from the sleep signals for controller **1212.** DDD **1210** may also include a drug delivery unit (DDU) **1216** for delivering therapeutic drug to a PD subject **1218.** Sleep monitoring system (SMS) **1214** may generally function in the same way or in a similar way as SMS **120** (Fig. 1) and/or SMS **280** (Fig. 2). Controller **1212** may control DDU **1216** in the same way or in a similar way as controller **150** controls DDU **140** (Fig. 1) and as controller **210** controls DDU **230** (Fig. 2).

Operational parameters calculation system (OPCS) **1220** may include a communication interface (the communication interface is not shown in Fig. 12) for receiving, for example, sleep data from remote controller **1212** and exchanging commands and other types of data with controller **1212** over a communication network **1230.** Operational parameters calculation system **1220** may also include an operational parameters calculator ("OPC") **1222** for calculating operational parameters values for controller **1212,** which are based on the sleep data that controller **1212** may transfer to OPCS **1220** over communication network **1230.** OPCS **1220** may also include a controller (the controller is not shown in Fig. 12) for operating OPC **1222** and for controlling, for example, inbound data transfer and outbound data transfer over communication network **1230.** Inbound data may include, for example, a message notifying OPCS **1220** that DDD **1210** is "On" and operating, sleep data that originate from SMS **1214,** and a "Request" for operational parameters values for DDD **1210** (e.g., for DDU **1216).** Outbound data may include, for example, a message from OPCS **1220** that acknowledges to DDD **1210** its "On" state. The outbound data may also include the operational parameters values requested by DDD **1210.**

An example way of operating DDS **1200** is described below. Controller **1212** may receive real time sleep data from SMS **1214,** or from a ('local') user interface (UI). (The UI, which is not shown in Fig. 12, may function in the same way or in a similar way as UI **160** and/or UI **240.)** Using communication network **1230,** controller **1212** may notify OPCS **1220** that DDD **1210** is "On" and operating, and it may also notify OPCS **1220** of its intention to transfer sleep data to OPCS **1220.** Controller **1212** may, then, send the sleep data to OPCS **1220** for processing, for example for calculating, or otherwise determining, operational parameters values for operating DDU **1216.** Controller **1212** may send to OPCS **1220** the sleep data together with a "Request" for OPCS **1220** to return the values for the operational parameters by which controller **1212** may control the operation of DDU **1216.**

OPCS **1220** may acknowledge to controller **1212** (via communication network **1230)** receiving the controller's (controller **1212)** notification and "Request" and receive the sleep data via communication network **1230.** Operational parameters calculator **1222** may, then, calculate, or otherwise determine, values for the operational parameters based on the sleep data. Then, OPCS **1220** may return the requested operational parameters values to controller **1212** via communication network **1230.** Upon receiving the requested values for the drug delivery operational parameters, controller **1212** may operate DDU **1216** to deliver the therapeutic drug to PD subject **1218** by using these operational parameters values.

Operational parameters calculator (OPC) **1222** may calculate, or otherwise determine, the operational parameters values for controller **1212** based, for example, on the sleep data, but also based on a predetermined drug delivery flow rate control scheme that may be adapted for (e.g., 'tailored' to) a specific PD subject. For example, while sleep data may provide information regarding the times (e.g., start time and duration) of the various sleep events (e.g., start time and duration of 'sleep stage 1' events, start time and duration of 'REM sleep' events, etc.), a drug delivery flow rate control scheme may specify the drug flow rate that is desired for each of the various sleep events, be it low or high, constant (for a period) or gradually changing (increasing at times and/or decreasing at other times).

Figs. 4 to 11 (inclusive), which are described herein, disclose example drug delivery flow rate control schemes that OPCS **1220** may use to calculate, or to otherwise determine, the operational parameters values. (Other drug delivery flow rate control schemes, which are useful for treating targeted conditions of specific PD subjects, may be used in a similar way.) A patient-specific drug delivery flow rate control scheme for implementation by DDD **1210** may be uploaded to OPCS **1220** locally, for example by using a user interface (the user interface is not shown in Fig. 12), or it may be transferred to OPCS **1220** over communication network **1230** from, for example, a sleep lab (or physician) **1240.** OPCS **1220** may use (e.g., compare) the uploaded, or transferred, drug delivery flow rate control scheme vis-à-vis the sleep data to calculate, or otherwise determine, the operational parameters values suitable for each sleep event. In some embodiments, a drug delivery flow rate control scheme may be sent from sleep lab (or physician) **1240** (for example) directly to controller **1212** (instead of to OPCS **1220)** for determining the operational parameters values by, for example, using (e.g., comparing) the drug delivery flow rate control scheme vis-à-vis the sleep data.

Communication network **1230** may be or include, for example, a wired network and/or a wireless communication network. The communication network may be, for example, the Internet, a Wi-Fi network, a Bluetooth network, wireless LAN (local area network), wireless WAN (wide area network), wireless MAN (metropolitan area network), a digital cellular network (e.g., GSM), and the like. Operational parameters calculation system (OPCS) **1220** and sleep lab **1240** may each be or include, for example, a wireless mobile device, a cellular telephone (for example smartphone), a personal digital assistant (PDA), a desktop computer, a laptop computer, and the like, that includes a suitable application and executes suitable algorithm(s).

According to some embodiments a drug delivery control scheme may be predetermined for treating ('targeting') a specific condition in a specific PD subject and remain unchanged for the entire sleep process. As described herein, aligning a drug delivery pattern (DDP) to sleep events associated with a PD subject (as shown, for example in Figs. 4, 5 and 6, and described herein) may be beneficial in treating a condition. So, if the alignment between the DDP and the sleep events is compromised some time during treatment (e.g., during sleep), the treatment may be less than optimal, if at all. In other embodiments, a drug delivery control scheme (DDCS) may initially be applied and modified later, in real time, during the day (e.g., during night) based on sleep data that may be collected or produced in real time. If real time sleep data indicate, or show, that there is misalignment between a currently used drug delivery pattern (DDP) and the real time sleep events, the DDCS may be modified (i.e., the relevant operational parameters values adjusted) in real time based on the real time sleep data, so that the DDP is realigned to the actual sleep events, and to the actual sleep pattern in general.

Sleep lab **1240** may be, or include, a personal computer, multi-processor systems, microprocessor-based or programmable consumer electronics, network PCs, minicomputers, mainframe computers, a hand-held device, a tablet, an iPad, a mobile phone, a smartphone such as an iPhone and an android phone, and the like, which can establish a connection, for example, via network **1230** (and/or via other communication networks) with DDD **1210** and OPCS **1220,** and, in general, with any server and/or cloud based application.

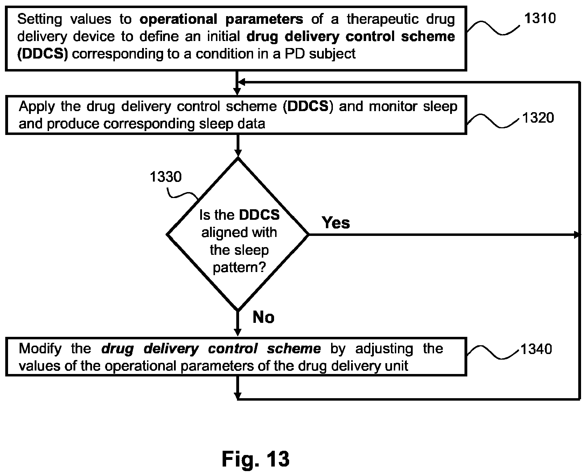

Fig. 13 shows a method of adjusting a drug delivery control scheme (DDCS) in real time in order to realign a drug delivery pattern (DDP) to sleep events as they occur, according to an example embodiment. At step 1310, a controller (e.g., controller **150, 210** or **1212)** may set initial values to operational parameters of a drug delivery unit (e.g., drug delivery unit **140, 230** or **1216)** to define an initial drug delivery control scheme (hence an initial drug delivery pattern). The initial drug delivery control scheme (DDCS) may be configured to target a specific condition in a specific PD subject. For example, the initial DDCS that the controller may use may resemble DDCS **520** (Fig. 5).

At step 1320, the controller may apply the initial DDCS, and, at a same time, a sleep monitoring system (SMS) (e.g., SMS **120, 280** or **1214)** may monitor the sleep of the PD subject while drug is delivered to the PD subject by using the initial DDCS. The SMS may produce sleep data that correspond to the monitored sleep, and the controller may process (e.g., parse, analyze) the sleep data to detect various sleep events in the sleep data, for example "sleep stage 1" event, "sleep stage 2" event, "REM sleep" event, "deep sleep" event, etc.

At step 1330, the controller may determine, for example by analyzing the initial DDCS vis-à-vis the detected sleep events, whether the initial DDCS is aligned with the detected sleep events. Referring again to Fig. 5, it demonstrates DDCS **520** aligned with the various sleep stages of sleep pattern **510.** For example, every time a REM sleep event is detected, the value of the drug flow rate level is high (e.g., **L1** in Fig. 5) for the duration of the REM sleep event, and low (e.g., **L2)** between REM sleep events. Since sleep patterns may change from one PD subject to another, and from one night to another, there may be a need to adjust the drug flow rate, timing and/or the duration in order to realign the DDCS to the actual sleep pattern. No matter which drug delivery control scheme the controller is using, the controller rechecks the alignment between the selected DDCS and the sleep pattern, and if a misalignment is detected, the controller adjusts the operational parameters to realign the DDCS to the sleep pattern.

Returning to step 1330, if the controller determines that the drug delivery control scheme (DDCS) is aligned with the actual sleep pattern (the condition is shown as **"Yes"** at step 1330), which means that the drug delivery is performed according to the plan, the initial DDCS remains unchanged and the subject's sleep is continued to be monitored (at step 1320). However, if the DDCS is not aligned with the actual sleep pattern, for example if the controller detects a mismatch between the DDCS and the sleep pattern (the condition is shown as **"No"** at step 1330), the controller may modify the DDCS by adjusting values of the operational parameters of the drug delivery unit (DDU) to more suitable values. The subject sleep's structure may, then, or concurrently, be continued to be monitored (at step 1320) to enable the controller to determine whether the DDCS requires additional adjustments during the remaining sleep period.

The articles "a" and "an" are used herein to refer to one or to more than one (e.g., to at least one) of the grammatical object of the article, depending on the context. By way of example, depending on the context, "an element" can mean one element or more than one element. The term "including" is used herein to mean, and is used interchangeably with, the phrase "including but not limited to". The terms "or" and "and" are used herein to mean, and are used interchangeably with, the term "and/or," unless context clearly indicates otherwise. The term "such as" is used herein to mean, and is used interchangeably, with the phrase "such as but not limited to".

Having thus described exemplary embodiments of the invention, it will be apparent to those skilled in the art that modifications of the disclosed embodiments will be within the scope of the invention. Alternative embodiments may, accordingly, include functionally equivalent objects/articles. Features of certain embodiments may be used with other embodiments shown herein. The present disclosure is relevant to (e.g., it may be implemented by, used with or for) various types of motor symptoms/disorders, non-motor symptoms/disorders, pumps, syringes, therapeutic drugs, infusion tubes/sets/lines, therapeutic drug dispensing devices, and the like. Hence the scope of the claims that follow is not limited by the disclosure herein.

Various aspects and embodiments of the present disclosure may further be understood with reference to the following numbered clauses:
1. A method of operating a therapeutic drug delivery system for treating a condition associated with a Parkinson's disease (PD) in a subject in need thereof, the method comprising:
   receiving, by the therapeutic drug delivery system, sleep data characterizing, or including, a chronologic sleep pattern (CSP) of the PD subject, and determining values for one or more operational parameters of the therapeutic drug delivery system based on the CSP, and/or
   receiving said values for the one or more operational parameters of the therapeutic drug delivery system; and
   operating the therapeutic drug delivery system according to the received operational parameters values or according to the determined operational parameters values to deliver a therapeutic drug composition to the PD subject.
2. The method as in clause 1, wherein the condition associated with PD is selected from the group consisting of: Parkinson symptom, motor complication, motor symptom, nonmotor symptom, and a sleep disorder.
3. The method as in clauses 1 or 2, wherein the values determined for the operational parameters and the values received for the operational parameters are optimized in terms of: optimizing sleep, improving sleep quality, ameliorating a sleep disorder, ameliorating a Parkinson symptom including tremor, shaking, slowed movement (bradykinesia), muscles rigidity, postural instability, walking/gait difficulties and dystonia; sleep duration, time interval from getting to bed to sleep onset time, number of awakenings during sleep, speed of body movement during sleep, time interval from awakening time until standing up, period of Parkinson "on" time versus "off time during wake time, awake time period during sleep, number of body rotations during sleep, average speed of rotation of body during sleep, average time of body rotation, degree of body rotation, or in terms of any combination thereof.
4. The method as in any of the preceding clauses, wherein the chronologic sleep pattern (CSP) comprises at least one of the following sleep events: sleep stage 1, sleep stage 2, sleep stage 3, rapid eye movement (REM) sleep, deep sleep stage, sleep cycle, wakeup time(s), sleep onset time and bedtime of the PD subject, and wherein a first operational parameter is a flow rate of the therapeutic drug composition and a second operational parameter is drug delivery timing for the therapeutic drug flow rate.
5. The method as in any of the preceding clauses, wherein operating the therapeutic drug delivery system comprises delivering the therapeutic drug composition at a flow rate that corresponds to delivering a low drug dose, or a gradually decreasing drug dose, or a high drug dose, or a gradually increasing drug dose, or a pulsating drug dose, or a continuous drug dose.
6. The method as in any of the preceding clauses, wherein the received values and the determined values of the operational parameters of the therapeutic drug delivery system define a drug delivery pattern (DDP).
7. The method as in clause 6, wherein the DDP corresponds to, adapted for, or derived from the chronologic sleep pattern (CSP).
8. The method as in any of the preceding clauses, wherein the sleep data comprises data regarding habitual bedtime, habitual sleep onset and habitual wakeup time(s) of the PD subject, and wherein the values of the operational parameters are determined based on one or more of the habitual bedtime, habitual sleep onset and/or habitual wakeup time.
9. The method as in any of the preceding clauses, wherein the sleep data comprises historical sleep data, said historical sleep data comprising at least historical sleep onset data and historical wakeup data, and wherein the values of the operational parameters are determined based on the historical sleep data, and wherein the historical sleep data comprises sleep data related to the PD subject and/or sleep data related to other PD subjects.
10. The method as in any of the preceding clauses, wherein values of the operational parameters are user-configured, pre-programmed, or determined based on output signals of one or more sensors.
11. The method as in any of the preceding clauses, wherein the treated condition is a sleep disorder, the sleep disorder selected from the group consisting of: Rapid Eye Movement sleep Behavior Disorder (RBD), REM sleep Without Atonia (RWA), Dream-Enactment Behavior (DEB), night wakefulness, insomnia (sleeplessness), sleep onset insomnia, daytime somnolence, number of awakenings during the night, sleep fragmentation, early-morning dystonia, akinesia, night atony, initiation of sleep, maintenance of sleep, daytime sudden sleep "attacks" episodes (narcolepsy), Excessive Daytime Sleepiness (EDS), hallucinations, impaired movement during sleep, difficulty turning in bed, NREM Stage III, REM sleep, sleep latency, wake after sleep onset time, impaired circadian rhythm (circadian rhythm disorder, abnormal 'sleep-wake' cycles), Slow Wave Sleep (SWS), Periodic Leg Movements in Sleep (PLMS).
12. The method as in any of the preceding clauses, wherein generating the sleep data is by a sleep monitoring system (SMS), by a wearable sleep sensor, and/or by using an electronic patient sleep diary.
13. The method as in any of clauses 6-12, comprising generating the sleep data and/or setting values to the operational parameters in real time according to the drug delivery pattern (DDP) that is to be applied to the PD subject.
14. The method as in any of the preceding clauses, wherein the therapeutic drug composition comprises a compound for treating or ameliorating a condition associated with PD.
15. The method as in clause 14, wherein the compound is selected from the group consisting of levodopa, a levodopa salt, a levodopa prodrug, a dopa decarboxylase inhibitor (DDI), a DDI salt, a DDI prodrug, a catechol-o-methyl-transferase (COMT) inhibitor, a COMT inhibitor salt, a COMT inhibitor prodrug, and any combination thereof.
16. The method as in clause15, wherein the DDI, salt, or prodrug thereof, is selected from the group consisting of carbidopa, benzaserazide, difluoromethyldopa, alpha-methyldopa, a salt thereof, a prodrug thereof, and any combination thereof.
17. The method as in any one of clauses 15 and 16, wherein the dose of each DDI is selected independently of any other DDI or active ingredients in the therapeutic drug.
18. The method as in any one of clauses 15-17, wherein the DDI is selected from the group consisting of carbidopa, a carbidopa salt, a carbidopa prodrug, and any combination thereof.
19. The method as in clause 14, wherein the therapeutic drug composition comprises at least one of levodopa, a levodopa salt, a levodopa prodrug, or any combination thereof.
20. The method as in clause 15, wherein the COMT inhibitor is selected from the group consisting of entacapone, tolcapone and any combination thereof.
21. The method as in clause 20, wherein the dose of each COMT inhibitor is selected independently of any other COMT inhibitor or active ingredients in the therapeutic drug.
22. The method as in any one of clauses 14-21, wherein the therapeutic drug composition comprises levodopa and carbidopa.
23. The method as in any one of clauses 14-22, wherein the therapeutic drug comprises composition levodopa, carbidopa and arginine.
24. The method as in any one of clauses 14-23, wherein the therapeutic drug composition comprises between about 4 % w/v to about 8 % w/v levodopa, between about 0.5 % w/v to about 1.0 % w/v carbidopa and between about 12 % w/v and about 17 % w/v arginine.
25. The method as in any one of clauses 14-21, wherein the therapeutic drug composition comprises a levodopa prodrug.
26. The method as in clause 25, wherein the levodopa prodrug is a levodopa-tyrosine conjugate.
27. The method as in any one of clauses 24 and 26, wherein the therapeutic drug composition further comprises carbidopa.
28. The method as in any one of clauses 24-27, wherein the therapeutic drug composition further comprises arginine.
29. The method as in any one of clauses 24-28, wherein the therapeutic drug composition comprises between about 20 % w/v and about 50 % w/v of a levodopa tyrosine conjugate, between about 0.5 % w/v and about 1.5 % carbidopa.
30. The method as in any one of clauses 14-29, wherein the therapeutic drug composition comprises levodopa, carbidopa and entacapone.
31. The method as in clause 30, wherein said therapeutic drug composition further comprises arginine.
32. The method as in any one of clauses 14-29, wherein the therapeutic drug composition comprises a levodopa prodrug, carbidopa, and entacapone.
33. The method as in clause 32, wherein said therapeutic drug composition further comprises arginine.
34. The method as in clause 32, wherein the levodopa prodrug is a levodopa tyrosine conjugate.
35. The method as in any one of clauses 24-34, wherein the therapeutic drug composition is substantially liquid at room temperature.
36. The method as in clause 4, further comprising identifying a REM sleep event in the chronologic sleep pattern (CSP) and adjusting values of the operational parameters to deliver the therapeutic drug at a high flow rate during the REM sleep event.
37. The method as in clause 4, further comprising identifying sleep cycles in the chronologic sleep pattern (CSP) and adjusting values of the operational parameters to deliver the therapeutic drug at a high flow rate after n sleep cycles (n=1, 2,...) are completed.
38. The method as in clause 4, comprising adjusting values of the operational parameters to deliver the therapeutic drug at low flow rate during deep sleep stage.
39. The method as in clause 7, wherein adjusting the values of the operational parameters is performed in real time to realign the drug delivery pattern (DDP) to sleep events included in the chronological sleep pattern (CSP).
40. The method as in any of the preceding clauses, wherein adjusting the values of the operational parameters is performed using Machine Learning.
41. A method of treating a subject suffering from Parkinson's Disease (PD) by a therapeutic drug delivery system, the method comprising:
   receiving, by the therapeutic drug delivery system, sleep data characterizing, or including, a chronologic sleep pattern (CSP) of a PD subject, and determining values for one or more operational parameters of the therapeutic drug delivery system based on the chronologic sleep pattern, and/or
   receiving values for the one or more operational parameters of the therapeutic drug delivery system; and
   operating the therapeutic drug delivery system according to the received operational parameters values and/or according to the determined operational parameters values to deliver a therapeutic compound to the PD subject.
42. The method as in clause 41, wherein the compound is selected from the group consisting of levodopa, a levodopa salt, a levodopa prodrug, a dopa decarboxylase inhibitor (DDI), a DDI salt, a DDI prodrug, a catechol-o-methyl-transferase (COMT) inhibitor, a COMT inhibitor salt, a COMT inhibitor prodrug, and any combination thereof.
43. The method as in clause 42, wherein the DDI, salt, or prodrug thereof, is selected from the group consisting of carbidopa, benzaserazide, difluoromethyldopa, alpha-methyldopa, a salt thereof, a prodrug thereof, and any combination thereof.
44. The method as in any one of clauses 42 and 43, wherein the dose of each DDI is selected independently of any other DDI or active ingredients in the therapeutic drug.
45. The method as in any one of clauses 42-44, wherein the DDI is selected from the group consisting of carbidopa, a carbidopa salt, a carbidopa prodrug, and any combination thereof.
46. The method as in clause 41, wherein the compound is selected from the group consisting of levodopa, a levodopa salt, a levodopa prodrug, or any combination thereof.
47. The method as in clause 42, wherein the COMT inhibitor is selected from entacapone, tolcapone and any combination thereof.
48. The method as in clause 47, wherein the dose of each COMT inhibitor is selected independently of any other COMT inhibitor or active ingredients in the compound.
49. A drug delivery system **(100,** Fig. 1; **200,** Fig. 2; **1200,** Fig. 12) for treating a condition associated with a subject with Parkinson's disease (PD), the drug delivery system comprising:
   a sleep data collecting unit (SDCU) for collecting and/or storing sleep data related to a PD subject and/or sleep data related to other PD subjects; and
   a drug delivery unit **(140, 230)** comprising:
      a drug reservoir **(142, 232)** for containing a therapeutic drug composition;
      a dispensing mechanism **(144, 234)** for dispensing the therapeutic drug composition from the drug reservoir to the subject; and
   a controller **(150, 210)** configured to:
      (i) receive values and/or determine values which are derived from the sleep data, for one or more drug delivery operational parameters of the dispensing mechanism; and
      (ii) operate the dispensing mechanism to deliver the therapeutic drug composition to the PD subject according to the values received or determined for the one or more operational parameters.
50. The system as in clause 49, wherein the treated condition is selected from the group consisting of: Parkinson symptom, motor complication, motor symptom, nonmotor symptom, and sleep disorder.
51. The system as in any of clauses 49 and 50, wherein the values determined for the operational parameters and the values received for the operational parameters are optimized in terms of: optimizing sleep, improving sleep quality, ameliorating a sleep disorder, ameliorating a Parkinson symptom including tremor, shaking, slowed movement (bradykinesia), muscles rigidity, postural instability, walking/gait difficulties and dystonia; sleep duration, time interval from getting to bed to sleep onset time, number of awakenings during sleep, speed of body movement during sleep, time interval from awakening time until standing up, period of Parkinson "on" time versus "off' time during wake time, awake time period during sleep, number of body rotations during sleep, average speed of rotation of body during sleep, average time of body rotation, degree of body rotation (degrees), or any combination thereof.
52. The system as in clause 49, wherein the sleep data collecting unit (SDCU) is configured in a configuration that is selected from at least one of:
   I. a first configuration in which the SDCU is or comprises a sleep monitoring system (SMS) **(120, 280),** the SMS comprising:
      a number ***n*** of sensors, the ***n*** sensors configured to produce signals associated, individually or collectively, with a sleep condition and/or motor activity of the PD subject (***n***=1, 2, 3,..., etc.), and
      a sensors interface **(132, 260)** connected to the ***n*** sensors, to receive the signals produced by ***n*** sensors and to convert the signals into sleep data; and
   II. a second configuration in which the SDCU comprises a user interface **(160, 240)** for manually uploading sleep data and/or a communication interface **(180)** for remotely uploading sleep data.
53. The system as in clause 49 wherein the controller is further configured to:
   (iii) detect, in the sleep data, a chronologic sleep pattern (CSP) of the subject; and
   (iv) deliver the therapeutic drug composition to the subject in a drug delivery pattern that corresponds to, adapted to or derived from the CSP.
54. The system as in clause 52, wherein at least one of the ***n*** sensors is user-wearable or otherwise attachable to the subject.
55. The system as in clause 52, wherein the ***n*** sensors are selected from the group consisting of: electrode, neuronal activity sensor, EEG sensor, ECG sensor, EMG sensor, polysomnography (PSG) sensor, sleep sensor, sleep state sensor, local field potential sensor, accelerometer, a wrist watch configured to detect sleep movements, an optical sensor, a camera, and any combination thereof.
56. The system as in clause 52, wherein the drug delivery system **(200)** comprises the sensors interface **(260).**
57. The system as in clause 52, wherein the one or more operational parameters of the dispensing mechanism are user-configurable or pre-programable by means of the user interface **(160, 240).**
58. The system as in clause 49, wherein the dispensing mechanism is configured to deliver the therapeutic drug composition to the PD subject intra-intestinally, subcutaneously, intravenously (IV), dermally, or by using a cerebrospinal fluid (CSF) pump.
59. The system as in clause 53, wherein the chronologic sleep pattern (CSP) comprises one or more of the following sleep events: sleep stage 1, sleep stage 2, sleep stage 3, rapid eye movement (REM) sleep, deep sleep stage, sleep cycle, wakeup time(s), sleep onset time and bedtime of the PD subject.
60. The system as in clause 49, wherein the operational parameters comprise a first operational parameter that is, or related to, a flow rate of the therapeutic drug composition, and a second operational parameter that is, or related to, drug delivering timing of the therapeutic drug composition.
61. The system as in clause 52, wherein the sleep data collecting unit (SDCU) is configured to collect, via the ***n*** sensors, data selected from the group consisting of representing movement of an upper limb (hand), representing movement of a lower limb (leg), representing head movement, representing hip movement, heart beat rate, breathing rate, Electromyography (EMG) signals, Electrocardiography (ECG) signals, Electroencephalography (EEG) signals, electrical potential signals, blood oxygen saturation level, skin conductivity and thorax movement.
62. The system as in clause 59, wherein the controller is configured to adjust values of the operational parameters to deliver the therapeutic drug composition at high flow rate during a REM sleep event.
63. The system as in clause 59, wherein the controller is configured to adjust values of the operational parameters to deliver the therapeutic drug composition at low flow rate during a deep sleep event.
64. The system as in clause 59, wherein the controller is configured to identify sleep cycles in the sleep data or in the chronologic sleep pattern (CSP) and to adjust values of the operational parameters to deliver the therapeutic drug composition at a high flow rate after 'n' sleep cycles are completed.
65. The system as in clause 49, wherein the controller is configured to deliver the therapeutic drug composition at a flow rate corresponding to an item selected from the group consisting of: delivering a low drug dose, delivering a gradually decreasing drug dose, delivering a high drug dose, delivering a gradually increasing drug dose, delivering a pulsating drug dose, and to delivering a continuous drug dose.
66. The system as in clause 49, wherein the controller is configured to adjust values of the operational parameters in real time to (re)align the drug delivery pattern (DDP) to sleep events included in the chronological sleep pattern (CSP).
67. The system as in clause 49, wherein the controller is configured to adjust values of the operational parameters by using Machine Learning.
68. The system as in clause 50, wherein the sleep disorder is selected from the group consisting of: Rapid Eye Movement sleep Behavior Disorder (RBD), REM sleep Without Atonia (RWA), Dream-Enactment Behavior (DEB), night wakefulness, insomnia (sleeplessness), sleep onset insomnia, daytime somnolence, number of awakenings, sleep fragmentation, early-morning dystonia, akinesia, night atony, initiation of sleep, maintenance of sleep, daytime sudden sleep "attacks" episodes (narcolepsy), Excessive Daytime Sleepiness (EDS), hallucinations, impaired movement during sleep, difficulty turning in bed, NREM Stage III, REM sleep, sleep latency, wake after sleep onset time, impaired circadian rhythm (circadian rhythm disorder, abnormal 'sleep-wake' cycles), Slow Wave Sleep (SWS), Periodic Leg Movements in Sleep (PLMS).
69. The system as in clause 50, wherein the therapeutic drug composition comprises a compound for treating or ameliorating the condition associated with the PD subject.
70. The system as in clause 69, wherein the compound is selected from the group consisting of levodopa, a levodopa salt, a levodopa prodrug, a dopa decarboxylase inhibitor (DDI), a DDI salt, a DDI prodrug, a catechol-o-methyl-transferase (COMT) inhibitor, a COMT inhibitor salt, a COMT inhibitor prodrug, and any combination thereof.
71. The system as in clause 70, wherein the DDI, salt, or prodrug thereof, is selected from the group consisting of carbidopa, benzaserazide, difluoromethyldopa, alpha-methyldopa, a salt thereof, a prodrug thereof, and any combination thereof.
72. The system as in any one of clauses 70 and 71, wherein the dose of each DDI is selected independently of any other DDI or active ingredients in the therapeutic drug composition.
73. The system as in any one of clauses 70-72, wherein the DDI is selected from the group consisting of carbidopa, a carbidopa salt, a carbidopa prodrug, and any combination thereof.
74. The system as in clause 69, wherein the compound is selected from the group consisting of levodopa, a levodopa salt, a levodopa prodrug, or any combination thereof.
75. The system as in clause 70, wherein the COMT inhibitor is selected from the group consisting of entacapone, tolcapone and any combination thereof.
76. The system as in clause 75, wherein the dose of each COMT inhibitor is selected independently of any other COMT inhibitor or active ingredients in the therapeutic drug composition.
77. The system as in any one of clauses 69-76, wherein the therapeutic drug composition comprises levodopa and carbidopa.
78. The system as in any one of clauses 69-77, wherein the therapeutic drug composition comprises levodopa, carbidopa and arginine.
79. The system as in any one of clauses 69-78, wherein the therapeutic drug composition comprises between about 4 % w/v to about 8 % w/v levodopa, between about 0.5 % w/v to about 1.0 % w/v carbidopa and between about 12 % w/v and about 17 % w/v arginine.
80. The system as in any one of clauses 69-76, wherein the therapeutic drug composition comprises a levodopa prodrug.
81. The system as in clause 80, wherein the levodopa prodrug is a levodopa-tyrosine conjugate.
82. The system as in any one of clauses 79 and 81, wherein the therapeutic drug composition further comprises carbidopa.
83. The system as in any one of clauses 79-82, wherein the therapeutic drug composition further comprises arginine.
84. The system as in any one of clauses 79-83, wherein the therapeutic drug composition comprises between about 20 % w/v and about 50 % w/v of a levodopa tyrosine conjugate, between about 0.5 % w/v and about 1.5 % carbidopa.
85. The system as in any one of clauses 69-84, wherein the therapeutic drug composition comprises levodopa, carbidopa and entacapone.
86. The system as in clause 85, wherein said therapeutic drug composition further comprises arginine.
87. The system as in any one of clauses 69-84, wherein the therapeutic drug composition comprises a levodopa prodrug, carbidopa, and entacapone.
88. The system as in clause 87, wherein said therapeutic drug composition further comprises arginine.
89. The system as in clause 87, wherein the levodopa prodrug is a levodopa tyrosine conjugate.
90. The system as in any one of clauses 69-89, wherein the therapeutic drug composition is substantially liquid at room temperature.

## Claims

1. A drug delivery system **(100,** Fig. 1; **200,** Fig. 2; **1200,** Fig. 12) for treating a condition associated with a subject with Parkinson's disease (PD), the drug delivery system comprising:
a sleep data collecting unit (SDCU) for collecting and/or storing sleep data related to a PD subject and/or sleep data related to other PD subjects; and
a drug delivery unit **(140, 230)** comprising:
a drug reservoir **(142, 232)** for containing a therapeutic drug composition;
a dispensing mechanism **(144, 234)** for dispensing the therapeutic drug composition from the drug reservoir to the subject; and
a controller **(150, 210)** configured to:
(i) receive values and/or determine values which are derived from the sleep data, for one or more drug delivery operational parameters of the dispensing mechanism; and
(ii) operate the dispensing mechanism to deliver the therapeutic drug composition to the PD subject according to the values received or determined for the one or more operational parameters.

2. The system as in claim 1, wherein the values determined for the operational parameters and the values received for the operational parameters are optimized in terms of: optimizing sleep, improving sleep quality, ameliorating a sleep disorder, ameliorating a Parkinson symptom including tremor, shaking, slowed movement (bradykinesia), muscles rigidity, postural instability, walking/gait difficulties and dystonia; sleep duration, time interval from getting to bed to sleep onset time, number of awakenings during sleep, speed of body movement during sleep, time interval from awakening time until standing up, period of Parkinson "on" time versus "off time during wake time, awake time period during sleep, number of body rotations during sleep, average speed of rotation of body during sleep, average time of body rotation, degree of body rotation (degrees), or any combination thereof.

3. The system as in claim 1 or 2, wherein the sleep data collecting unit (SDCU) is configured in a configuration that is selected from at least one of:
A. a first configuration in which the SDCU is or comprises a sleep monitoring system (SMS) **(120, 280),** the SMS comprising:
a number ***n*** of sensors, the ***n*** sensors configured to produce signals associated, individually or collectively, with a sleep condition and/or motor activity of the PD subject (***n***=1, 2, 3,..., etc.), and
a sensors interface **(132, 260)** connected to the ***n*** sensors, to receive the signals produced by ***n*** sensors and to convert the signals into sleep data; and
B. a second configuration in which the SDCU comprises a user interface **(160, 240)** for manually uploading sleep data and/or a communication interface **(180)** for remotely uploading sleep data.

4. The system as in any preceding claim, wherein the controller is further configured to:
(iii) detect, in the sleep data, a chronologic sleep pattern (CSP) of the subject; and
(iv) deliver the therapeutic drug composition to the subject in a drug delivery pattern that corresponds to, adapted to or derived from the CSP.

5. The system as in claim3, wherein at least one of the ***n*** sensors is user-wearable or otherwise attachable to the subject; and/or wherein the ***n*** sensors are selected from the group consisting of: electrode, neuronal activity sensor, EEG sensor, ECG sensor, EMG sensor, polysomnography (PSG) sensor, sleep sensor, sleep state sensor, local field potential sensor, accelerometer, a wrist watch configured to detect sleep movements, an optical sensor, a camera, and any combination thereof.

6. The system as in claim 4, wherein the chronologic sleep pattern (CSP) comprises one or more of the following sleep events: sleep stage 1, sleep stage 2, sleep stage 3, rapid eye movement (REM) sleep, deep sleep stage, sleep cycle, wakeup time(s), sleep onset time and bedtime of the PD subject;
optionally:
wherein the controller is configured to adjust values of the operational parameters to deliver the therapeutic drug composition at high flow rate during a REM sleep event; or
wherein the controller is configured to adjust values of the operational parameters to deliver the therapeutic drug composition at low flow rate during a deep sleep event; or
wherein the controller is configured to identify sleep cycles in the sleep data or in the chronologic sleep pattern (CSP) and to adjust values of the operational parameters to deliver the therapeutic drug composition at a high flow rate after 'n' sleep cycles are completed.

7. The system as in claim 4, wherein the sleep data collecting unit (SDCU) is configured to collect, via the n sensors, data selected from the group consisting of representing movement of an upper limb (hand), representing movement of a lower limb (leg), representing head movement, representing hip movement, heart beat rate, breathing rate, Electromyography (EMG) signals, Electrocardiography (ECG) signals, Electroencephalography (EEG) signals, electrical potential signals, blood oxygen saturation level, skin conductivity and thorax movement.

8. The system as in any preceding claim, wherein the controller is configured to deliver the therapeutic drug composition at a flow rate corresponding to an item selected from the group consisting of: delivering a low drug dose, delivering a gradually decreasing drug dose, delivering a high drug dose, delivering a gradually increasing drug dose, delivering a pulsating drug dose, and to delivering a continuous drug dose; and/or
wherein the controller is configured to adjust values of the operational parameters in real time to (re)align the drug delivery pattern (DDP) to sleep events included in the chronological sleep pattern (CSP); and/or
wherein the controller is configured to adjust values of the operational parameters by using Machine Learning.

9. The system as in any preceding claim, wherein the treated condition is selected from the group consisting of: Parkinson symptom, motor complication, motor symptom, nonmotor symptom, and sleep disorder;
optionally wherein the sleep disorder is selected from the group consisting of: Rapid Eye Movement sleep Behavior Disorder (RBD), REM sleep Without Atonia (RWA), Dream-Enactment Behavior (DEB), night wakefulness, insomnia (sleeplessness), sleep onset insomnia, daytime somnolence, number of awakenings, sleep fragmentation, early-morning dystonia, akinesia, night atony, initiation of sleep, maintenance of sleep, daytime sudden sleep "attacks" episodes (narcolepsy), Excessive Daytime Sleepiness (EDS), hallucinations, impaired movement during sleep, difficulty turning in bed, NREM Stage III, REM sleep, sleep latency, wake after sleep onset time, impaired circadian rhythm (circadian rhythm disorder, abnormal 'sleep-wake' cycles), Slow Wave Sleep (SWS), Periodic Leg Movements in Sleep (PLMS).

10. The system as in claim 9, wherein the therapeutic drug composition comprises a compound for treating or ameliorating the condition associated with the PD subject;
optionally wherein the compound is selected from the group consisting of levodopa, a levodopa salt, a levodopa prodrug, a dopa decarboxylase inhibitor (DDI), a DDI salt, a DDI prodrug, a catechol-o-methyl-transferase (COMT) inhibitor, a COMT inhibitor salt, a COMT inhibitor prodrug, and any combination thereof;
optionally wherein the DDI, salt, or prodrug thereof, is selected from the group consisting of carbidopa, benzaserazide, difluoromethyldopa, alpha-methyldopa, a salt thereof, a prodrug thereof, and any combination thereof;
optionally wherein the dose of each DDI is selected independently of any other DDI or active ingredients in the therapeutic drug composition;
optionally wherein the DDI is selected from the group consisting of carbidopa, a carbidopa salt, a carbidopa prodrug, and any combination thereof;
optionally wherein the compound is selected from the group consisting of levodopa, a levodopa salt, a levodopa prodrug, or any combination thereof;
optionally wherein the COMT inhibitor is selected from the group consisting of entacapone, tolcapone and any combination thereof;
optionally wherein the dose of each COMT inhibitor is selected independently of any other COMT inhibitor or active ingredients in the therapeutic drug composition;
optionally wherein the therapeutic drug composition comprises levodopa and carbidopa;
optionally wherein the therapeutic drug composition comprises levodopa, carbidopa and arginine;
optionally wherein the therapeutic drug composition comprises between about 4 % w/v to about 8 % w/v levodopa, between about 0.5 % w/v to about 1.0 % w/v carbidopa and between about 12 % w/v and about 17 % w/v arginine;
optionally wherein the therapeutic drug composition comprises a levodopa prodrug;
optionally wherein the levodopa prodrug is a levodopa-tyrosine conjugate;
optionally wherein the therapeutic drug composition further comprises carbidopa;
optionally wherein the therapeutic drug composition further comprises arginine;
optionally wherein the therapeutic drug composition comprises between about 20 % w/v and about 50 % w/v of a levodopa tyrosine conjugate, between about 0.5 % w/v and about 1.5 % carbidopa;
optionally wherein the therapeutic drug composition comprises levodopa, carbidopa and entacapone;
optionally wherein the therapeutic drug composition comprises a levodopa prodrug, carbidopa, and entacapone;
optionally wherein the therapeutic drug composition is substantially liquid at room temperature.

11. A method of operating a therapeutic drug delivery system for treating a condition associated with a Parkinson's disease (PD) in a subject in need thereof, the method comprising:
receiving, by the therapeutic drug delivery system, sleep data characterizing, or including, a chronologic sleep pattern (CSP) of the PD subject, and determining values for one or more operational parameters of the therapeutic drug delivery system based on the CSP, and/or
receiving said values for the one or more operational parameters of the therapeutic drug delivery system; and
operating the therapeutic drug delivery system according to the received operational parameters values or according to the determined operational parameters values to dose therapeutic drug composition for delivery to the PD subject.

12. The method as in claim 11, wherein the values determined for the operational parameters and the values received for the operational parameters are optimized in terms of: optimizing sleep, improving sleep quality, ameliorating a sleep disorder, ameliorating a Parkinson symptom including tremor, shaking, slowed movement (bradykinesia), muscles rigidity, postural instability, walking/gait difficulties and dystonia; sleep duration, time interval from getting to bed to sleep onset time, number of awakenings during sleep, speed of body movement during sleep, time interval from awakening time until standing up, period of Parkinson "on" time versus "off time during wake time, awake time period during sleep, number of body rotations during sleep, average speed of rotation of body during sleep, average time of body rotation, degree of body rotation, or in terms of any combination thereof.

13. The method as in claim 11 or 12, wherein the chronologic sleep pattern (CSP) comprises at least one of the following sleep events: sleep stage 1, sleep stage 2, sleep stage 3, rapid eye movement (REM) sleep, deep sleep stage, sleep cycle, wakeup time(s), sleep onset time and bedtime of the PD subject, and wherein a first operational parameter is a flow rate of the therapeutic drug composition and a second operational parameter is drug delivery timing for the therapeutic drug flow rate.

14. The method as in any of claims 11 to 13, wherein the received values and the determined values of the operational parameters of the therapeutic drug delivery system define a drug delivery pattern (DDP);
optionally wherein the DDP corresponds to, is adapted for, or is derived from the chronologic sleep pattern (CSP).

15. The method as in any one of claims 11 to 14, wherein the sleep data comprises data regarding habitual bedtime, habitual sleep onset and habitual wakeup time(s) of the PD subject, and wherein the values of the operational parameters are determined based on one or more of the habitual bedtime, habitual sleep onset and habitual wakeup time; and/or
wherein the sleep data comprises historical sleep data, said historical sleep data comprising at least historical sleep onset data and historical wakeup data, and wherein the values of the operational parameters are determined based on the historical sleep data, and wherein the historical sleep data comprises sleep data related to the PD subject and/or sleep data related to other PD subjects.

16. The method as in any one of claims 11 to 15, wherein the condition associated with PD is selected from the group consisting of: Parkinson symptom, motor complication, motor symptom, nonmotor symptom, and a sleep disorder; and/or
wherein the treated condition is a sleep disorder, the sleep disorder selected from the group consisting of: Rapid Eye Movement sleep Behavior Disorder (RBD), REM sleep Without Atonia (RWA), Dream-Enactment Behavior (DEB), night wakefulness, insomnia (sleeplessness), sleep onset insomnia, daytime somnolence, number of awakenings during the night, sleep fragmentation, early-morning dystonia, akinesia, night atony, initiation of sleep, maintenance of sleep, daytime sudden sleep "attacks" episodes (narcolepsy), Excessive Daytime Sleepiness (EDS), hallucinations, impaired movement during sleep, difficulty turning in bed, NREM Stage III, REM sleep, sleep latency, wake after sleep onset time, impaired circadian rhythm (circadian rhythm disorder, abnormal 'sleep-wake' cycles), Slow Wave Sleep (SWS), Periodic Leg Movements in Sleep (PLMS).

17. The method as in any of claims 11 to 16, wherein generating the sleep data is by a sleep monitoring system (SMS), by a wearable sleep sensor, and/or by using an electronic patient sleep diary.

18. The method as in any one of claims 11 to 18, wherein the therapeutic drug composition comprises a compound for treating or ameliorating a condition associated with PD;
optionally wherein the compound is selected from the group consisting of levodopa, a levodopa salt, a levodopa prodrug, a dopa decarboxylase inhibitor (DDI), a DDI salt, a DDI prodrug, a catechol-o-methyl-transferase (COMT) inhibitor, a COMT inhibitor salt, a COMT inhibitor prodrug, and any combination thereof;
optionally wherein the DDI, salt, or prodrug thereof, is selected from the group consisting of carbidopa, benzaserazide, difluoromethyldopa, alpha-methyldopa, a salt thereof, a prodrug thereof, and any combination thereof;
optionally wherein the dose of each DDI is selected independently of any other DDI or active ingredients in the therapeutic drug composition;
optionally wherein the DDI is selected from the group consisting of carbidopa, a carbidopa salt, a carbidopa prodrug, and any combination thereof;
optionally wherein the therapeutic drug composition comprises at least one of levodopa, a levodopa salt, a levodopa prodrug, or any combination thereof;
optionally wherein the COMT inhibitor is selected from the group consisting of entacapone, tolcapone and any combination thereof;
optionally wherein the dose of each COMT inhibitor is selected independently of any other COMT inhibitor or active ingredients in the therapeutic drug composition;
optionally wherein the therapeutic drug composition comprises levodopa and carbidopa;
optionally wherein the therapeutic drug composition comprises levodopa, carbidopa and arginine;
optionally wherein the therapeutic drug composition comprises between about 4 % w/v to about 8 % w/v levodopa, between about 0.5 % w/v to about 1.0 % w/v carbidopa and between about 12 % w/v and about 17 % w/v arginine;
optionally wherein the levodopa prodrug is a levodopa-tyrosine conjugate;
optionally wherein the therapeutic drug composition further comprises carbidopa;
optionally wherein the therapeutic drug composition further comprises arginine;
optionally wherein the therapeutic drug composition comprises between about 20 % w/v and about 50 % w/v of a levodopa tyrosine conjugate, between about 0.5 % w/v and about 1.5 % carbidopa;
optionally wherein the therapeutic drug composition comprises levodopa, carbidopa and entacapone;
optionally wherein the therapeutic drug composition comprises a levodopa prodrug, carbidopa, and entacapone;
optionally wherein the therapeutic drug composition is substantially liquid at room temperature.

19. The method as in claim 13, further comprising identifying a REM sleep event in the chronologic sleep pattern (CSP) and adjusting values of the operational parameters to deliver the therapeutic drug composition at a high flow rate during the REM sleep event; and/or
further comprising identifying sleep cycles in the chronologic sleep pattern (CSP) and adjusting values of the operational parameters to deliver the therapeutic drug composition at a high flow rate after ***n*** sleep cycles (n=1, 2,...) are completed; and/or
further comprising adjusting values of the operational parameters to deliver the therapeutic drug composition at low flow rate during deep sleep stage.
